(19) Europäisches Patentamt
European Patent Office
Office européen des brevets

(11) Veröffentlichungsnummer: **0 316 582 B1**

(12) # EUROPÄISCHE PATENTSCHRIFT

(45) Veröffentlichungstag der Patentschrift: **30.09.92**

(51) Int. Cl.[5]: **C07D 211/58**, C07D 401/12, C07D 405/12, C08K 5/34

(21) Anmeldenummer: **88117167.2**

(22) Anmeldetag: **15.10.88**

Die Akte enthält technische Angaben, die nach dem Eingang der Anmeldung eingereicht wurden und die nicht in dieser Patentschrift enthalten sind.

(54) **4-Formylaminopiperidinderivate, deren Verwendung als Stabilisatoren und damit stabilisiertes organisches Material.**

(30) Priorität: **14.11.87 DE 3738736**

(43) Veröffentlichungstag der Anmeldung:
**24.05.89 Patentblatt 89/21**

(45) Bekanntmachung des Hinweises auf die Patenterteilung:
**30.09.92 Patentblatt 92/40**

(84) Benannte Vertragsstaaten:
**BE CH DE ES FR GB IT LI NL SE**

(56) Entgegenhaltungen:
**DE-A- 2 349 962**
**US-A- 4 191 683**

(73) Patentinhaber: **BASF Aktiengesellschaft**
**Carl-Bosch-Strasse 38**
**W-6700 Ludwigshafen(DE)**

(72) Erfinder: **Aumueller, Alexander, Dr.**
**An der Marlach 5**
**W-6705 Deidesheim(DE)**
Erfinder: **Neumann, Peter, Dr.**
**Franz-Schubert-Strasse 1**
**W-6908 Wiesloch(DE)**
Erfinder: **Trauth, Hubert**
**Milanstrasse 5**
**W-6724 Dudenhofen(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Es ist bekannt, daß 2,2,6,6-Tetraalkyl-piperidinderivate Lichtschutzmittel für organische Polymere sind. Unbefriedigend ist oft die Verträglichkeit mit Polyolefinen und anderen Kunststoffen, die Dauer der Schutzwirkung, die Eigenfarbe der Substanzen und die thermische Zersetzung der Stabilisatoren beim Einarbeiten in Polymere bei erhöhter Temperatur.

In der DE-PS 2 349 962 werden Tetraalkylpiperidinderivate der Formel

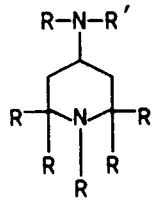

beschrieben, worin R' u.a. auch eine Acylgruppe mit 2 bis 18 Kohlenstoffen darstellt. Die Verbindungen wurden zum Stabilisieren von Polymeren vorgeschlagen.

Der Erfindung liegt die Aufgabe zugrunde, neue Polyalkylpiperidinderivate zur Verfügung zu stellen, welche die vorstehenden Nachteile nicht aufweisen.

Diese Aufgabe wird mit Hilfe der Polyalkylpiperidinverbindungen der Erfindung gelöst.

Dementsprechend betrifft die Erfindung 4-Formylaminopiperidinderivate der allgemeinen Formel I

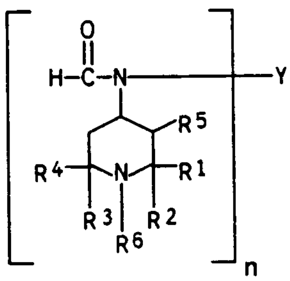

(I),

in der

| | |
|---|---|
| n | 1 oder 2, |
| $R^1$, $R^2$, $R^3$ und $R^4$ | unabhängig voneinander $C_1$- bis $C_4$-Alkyl oder |
| $R^1$ und $R^2$ oder $R^3$ und $R^4$ | zusammen eine Tetramethylen- oder Pentamethylengruppe, |
| $R^5$ | Wasserstoff oder $C_1$- bis $C_4$-Alkyl, |
| $R^6$ | Wasserstoff, $C_1$- bis $C_{22}$-Alkyl, $C_3$- bis $C_{22}$-Alkenyl, gegebenenfalls durch $C_1$-$C_4$-Alkyl, Fluor, Chlor, $C_1$-$C_4$-Alkoxy, Methylendioxy, Ethylendioxy und/oder Di-$C_1$-$C_4$-alkylamino substituiertes $C_7$- bis $C_{12}$-Phenylalkyl, $C_1$- bis $C_{22}$-C-Alkanoyl, $C_2$- bis $C_3$-Cyanalkyl, $C_1$- bis $C_{22}$-Hydroxyalkyl oder $C_2$- bis $C_{22}$-Aminoalkyl und |

- wenn n = 1 ist -

Y Wasserstoff, $C_1$- bis $C_{22}$-Alkyl, $C_3$- bis $C_{22}$-Alkenyl, $C_3$- bis $C_{12}$-Cycloalkyl oder Bicycloalkyl, durch Cyan, Hydroxy oder Carbo-$C_1$-$C_4$-alkoxy substituiertes $C_2$- bis $C_{22}$-Alkyl, durch Ethersauerstoff, Stickstoff oder Schwefel unterbrochenes $C_4$-$C_{22}$-Alkyl, gegebenenfalls durch $C_1$-$C_4$-Alkyl, Fluor, Chlor, $C_1$-$C_4$-Alkoxy, Methylendioxy, Ethylendioxy oder Di-$C_1$-$C_4$-alkylamino substituiertes $C_7$- bis $C_{22}$-Phenyl- oder Diphenylalkyl, gegebenenfalls durch $C_1$-$C_4$-Alkyl oder Carbo-$C_1$-$C_4$-alkoxy substituiertes Phenyl, ein Rest der Formel

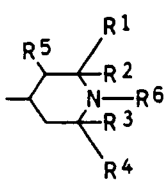

oder heterocyclische Reste der Formel

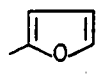 , 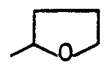 , 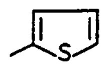 , 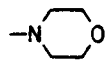 , 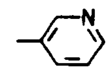

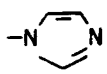 , 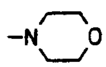 , 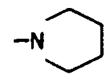 oder 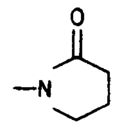

enthaltendes $C_1$-$C_{22}$-Alkyl, oder

- wenn n = 2 ist -

Y $C_2$- bis $C_{22}$-Alkylen, $C_5$- bis $C_{22}$-Cycloalkylen, $C_8$- bis $C_{14}$-Phenylalkylen, Phenylen oder durch Ethersauerstoff, Stickstoff, Schwefel oder 1,4-Piperazinylen unterbrochenes $C_4$- bis $C_{30}$-Alkylen bedeuten,

sowie die Säureadditionssalze dieser Verbindungen.

Die erfindungsgemäßen Verbindungen besitzen außerordentlich gute stabilisierende Eigenschaften, haben keine Eigenfarbe, sind gut verträglich mit organischen Polymeren, Zeigen einen niedrigen Dampfdruck und sind stabil gegen thermische Zersetzung.

Bevorzugt stehen $R^1$ bis $R^4$ für Methyl. Für $R^5$ ist Wasserstoff bevorzugt.

Für $R^6$ kommen außer Wasserstoff, z.B. im einzelnen in Betracht: Methyl, Ethyl, Propyl, Butyl, Pentyl, Hexyl; Benzyl, Phenylethyl, Phenylpropyl, Methylbenzyl; Allyl; Acetyl, Propionyl, Butanoyl, Pentanoyl, Benzoyl; Cyanmethyl, Hydroxyethyl und Aminoethyl.

Für $R^6$ sind Methyl, Acetyl, Cyanmethyl, Aminoethyl und insbesondere Wasserstoff bevorzugt.

Für Y sind außer Wasserstoff z.B. zu nennen:

a) $C_1$-$C_{22}$-Alkyl, wie Methyl, Ethyl, n- und i-Propyl, n- und i-Butyl, n- und i-Pentyl, Hexyl, Octyl, Decyl, Dodecyl, Octadecyl, Pivalyl, 3,3-Dimethylbutyl-2, Neopentyl, 4-Methylpentyl-2 und 2-Ethylhexyl;

b) $C_3$-$C_{22}$-Alkenyl wie Allyl, Butenyl, Pentenyl und Oleyl;

c) $C_3$-$C_{12}$-Cycloalkyl wie Cyclopropyl, Cyclobutyl, Cyclopentyl, Cyclohexyl, Methylcyclohexyl, Cycloheptyl, Cyclooctyl, Cyclododecyl und Bicycloheptyl, von denen Cyclopentyl und Cyclohexyl bevorzugt sind;

d) durch Cyan, Hydroxy oder Carboalkoxy substituiertes $C_2$-$C_{22}$-Alkyl wie Cyanmethyl, Hydroxyethyl, Hydroxypropyl, Hydroxybutyl, Carbomethoxyethyl und Carboethoxyethyl;

e) durch Ethersauerstoff oder Stickstoff unterbrochenes und gegebenenfalls durch Hydroxy substituiertes $C_4$-$C_{22}$-Alkyl wie $-(CH_2)_3N(CH_3)_2$, $-(CH_2)_3N(C_2H_5)_2$, $-(CH_2)_3-OCH_3$, $-(CH_2)_3-O-CH(CH_3)_2$, $-(CH_2)_2O(CH_2)_2-OH$, $-CH_2-(CH_2)_2-CH_2-N(CH_2)_3$, $-(CH_2)_2-N[CH(CH_3)_2]_2$, $-(CH_2)_2-N(C_2H_5)_2$, $-(CH_2)_2N(CH_3)_2$, $-(CH_2)_2OCH_3$ und $-(CH_2)_2OCH_2CH_3$;

f) gegebenenfalls substituiertes $C_7$-$C_{22}$-Phenyl- und Diphenylalkyl wie Benzyl, Methoxybenzyl, Methylbenzyl, Ethylbenzyl, Isopropylbenzyl, Trimethylbenzyl, Fluorbenzyl, Chlorbenzyl, Methylendioxybenzyl, Phenylethyl, Phenylpropyl und Phenylbutyl, Dimethylaminobenzyl, Diphenylmethyl und 1,3-Diphenylpropyl-2;

g) gegebenenfalls substituiertes Phenyl wie Phenyl, Tolyl und durch Carbo-$C_1$-$C_4$-alkoxy substituiertes Phenyl;

h) Heterocyclische Reste der Formel

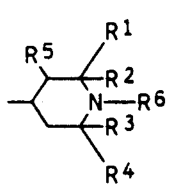

worin $R^1$ bis $R^6$ die obengenannte Bedeutung haben;

i) Heterocylen enthaltendes $C_1$-$C_{22}$-Alkyl wie

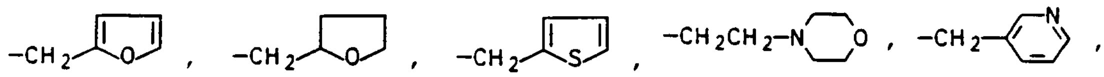

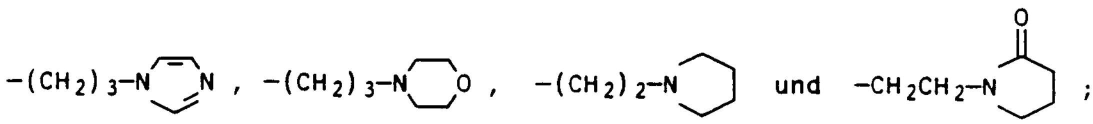

und ;

k) $C_2$-$C_{22}$-Alkylen- und $C_5$-$C_{22}$-Cycloalkylen wie $-(CH_2)_o-CH_2-$ (mit o = 1 bis 21),

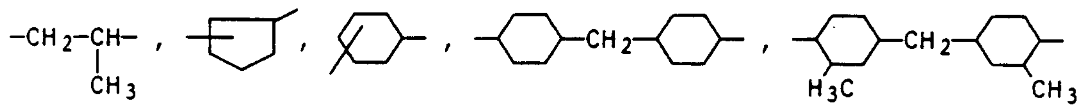

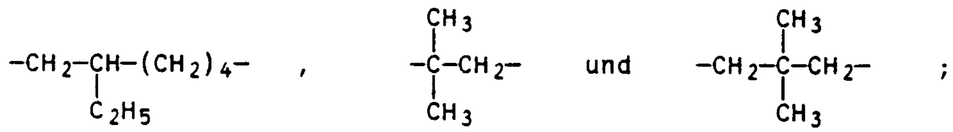

und ;

l) $C_8$-$C_{14}$-Phenylalkylen und Phenylen wie

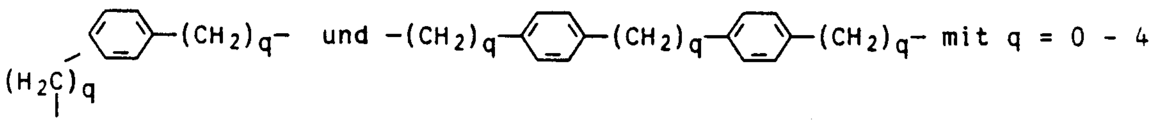

und mit q = 0 - 4

m) durch Ethersauerstoff, Stickstoff oder 1,4-Piperazinylen unterbrochenes Alkylen wie

$-(CH_2)_3O(CH_2)_4O(CH_2)_3-$ , $-(CH_2)_3O(CH_2)_2O(CH_2)_2O(CH_2)_3-$ ,

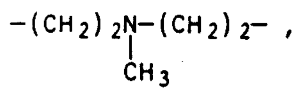 , $-(C_3H_6O)_r-C_3H_6-$ mit r = 1 bis 33,

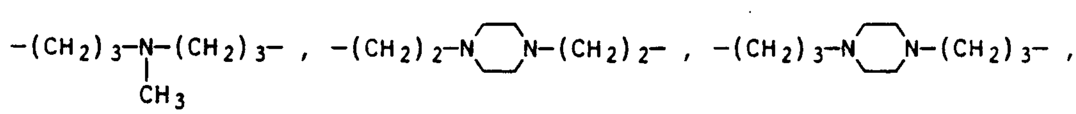

$-(CH_2)_3O(CH_2)_2O(CH_2)_3-$ , 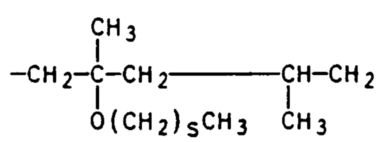 ,

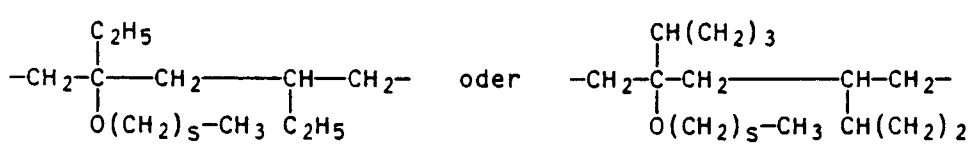

oder

mit s = 0 bis 7.

Verbindungen der allgemeinen Formel (I) können durch Reaktion von Verbindungen der allgemeinen Formel (II) mit Ameisensäure oder Ameisensäureestern hergestellt werden. Hierfür sind der Methyl- und der Ethylester bevorzugt. Dabei kann mit oder ohne Katalysator gearbeitet werden. Katalysatoren können dabei Lewissäuren sein, von denen besonders Titanorthoester und hier speziell Titanorthobutylat genannt sein

sollen.

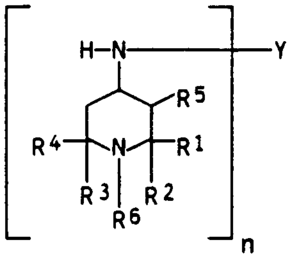

(II)

Verbindungen der allgemeinen Formel (I) mit $R^6$ = H können nach an sich bekannten Verfahren wie Alkylierung, reduktive Aminierung, Umsetzung mit Glykolsäurenitril u. a. in Verbindungen der allgemeinen Formel (I) mit R ‡ H umgewandelt werden.

Die erfindungsgemäßen Verbindungen können in Form der freien Basen oder als Salze vorliegen. Geeignete Anionen stammen z. B. von anorganischen Säuren und insbesondere von organischen Carbonsäuren sowie organischen Sulfonsäuren.

Als anorganische Anionen sind z.B. Chlorid, Bromid, Sulfat, Methosulfat, Tetrafluoroborat, Phosphat und Rhodanid zu nennen.

Als Carbonsäureanionen kommen z.B. Formiat, Acetat, Propionat, Hexanoat, Cyclohexanoat, Lactat, Stearat, Dodecylbenzoat, Benzoat, Acrylat, Methacrylat, Citrat, Malonat oder Succinat sowie Anionen von Polycarbonsäuren mit bis zu 3000 COOH-Gruppen in Betracht.

Sulfonsäure-Anionen sind beispielsweise Benzolsulfonat oder Tosylat.

Die erfindungsgemäßen Verbindungen eignen sich zum Stabilisieren von organischem Material, speziell von Kunststoffen, gegen den Abbau durch Licht und Wärme. Sie sind auch wirksam als Metalldesaktivator. Sie werden den zu stabilisierenden Kunststoffen in einer Konzentration von 0,01 bis 5 Gew.-%, vorzugsweise von 0,02 bis 1 Gew.-%, bezogen auf das Polymere, vor, während oder nach der Polymerbildung zugesetzt.

Zur Vermischung der erfindungsgemäßen Verbindungen mit den Kunststoffen können alle bekannten Vorrichtungen und Methoden zum Einmischen von Stabilisierungsmitteln oder anderen Zusätzen in Polymere angewandt werden.

Die durch die erfindungsgemäßen Verbindungen stabilisierten Kunststoffe können gegebenenfalls noch weitere Additive enthalten, z.B. Antioxidantien, Lichtstabilisierungsmittel, Metalldesaktivatoren, antistatische Mittel, flammhemmende Mittel, Pigmente und Füllstoffe.

Antioxidantien und Lichtstabilisatoren, die den Kunststoffen neben den erfindungsgemäßen Verbindungen zugesetzt werden können, sind z.B. Verbindungen auf der Basis sterisch gehinderter Phenole oder Schwefel oder Phosphor enthaltende Costabilisatoren.

Als derartige phenolische Antioxidationsmittel seien beispielsweise 2,6-Di-tert.-butyl-4-methylphenol, n-Octadecyl-$\beta$-(3,5-di-tert.-butyl-4-hydroxyphenyl)-propionat, 1,1,3-Tris-(2-methyl-4-hydroxy-5-tert.-butylphenyl)-butan, 1,3,5-Trimethyl-2,4,6-tris-(3,5-di-tert.-butyl-4-hydroxybenzyl)-benzol, 1,3,5-Tris-(3,5-di-tert.-butyl-4-hydroxybenzyl)-isocyanurat, 1,3,5-Tris-[$\beta$-(3,5-di-tert.-butyl-4-hydroxyphenyl)-propionyloxyethyl]-iso cyanurat, 1,3,5-Tris-(2,6,dimethyl-3-hydroxy-4-tert.-butylbenzyl)-isocyanurat und Pentaerythrit-tetrakis-[$\beta$-(3,5-di-tert.-butyl-4-hydroxy-phenyl)-propionat] erwähnt.

Als phosphorhaltige Antioxidantien kommen beispielsweise Tris-(nonylphenyl-phosphit, Distearylpentaerythritdiphosphit, Tris-(2,4-di-tert.-butyl-phenyl)-phosphit, Tris-(2-tert.-butyl-4-methylphenyl)-phosphit, Bis-(2,4-di-tert.-butylphenyl)-pentaerythritdiphosphit und Tetrakis-(2,4-di-tert.-butylphenyl)-4,4'-biphenylendiphosphit in Betracht.

Als Schwefel enthaltende Antioxidationsmittel seien beispielsweise Dilaurylthiodipropionat, Dimyristylthiodipropionat, Distearylthiodipropionat, Pentaerythrittetrakis-($\beta$-laurylthiopropionat) und Pentaerythrittetrakis-($\beta$-laurylthiopropionat) und Pentaerythrittetrakis-($\beta$-laurylthiopropionat) und Pentaerythrittetrakis-($\beta$-hexylthiopropionat) genannt.

Weitere Antioxidantien und Lichtstabilisatoren, die zusammen mit den erfindungsgemäßen Verbindungen verwendet werden können, sind z.B. 2-(2'-Hydroxyphenyl)-benztriazole, 2-Hydroxybenzophenone, Arylester von Hydroxybenzoesäuren, $\alpha$-Cyanozimtsäurederivate, Nickelverbindungen oder Oxalsäuredianilide.

Als organische Polymere, die durch die erfindungsgemäßen Verbindungen stabilisiert werden können, seien beispielsweise genannt:

Polymere von Mono- und Diolefinen, wie z. B. Polyethylen niedriger oder hoher Dichte, lineares Polybuten-1, Polyisopren, Polybutadien sowie Copolymerisate von Mono- oder Diolefinen oder Mischungen der genannten Polymeren;
Copolymerisate von Mono- oder Diolefinen mit anderen Vinylmonomeren wie z.B. Ethylen-Alkylacrylat-Copolymere, Ethylen-Alkylmethacrylat-Copolymere, Ethylen-Vinylacetat-Copolymere oder Ethylen-Acrylsäure-Copolymere;

Polystyrol;

Copolymere von Styrol oder $\alpha$-Methylstyrol mit Dienen oder Acrylderivaten, wie z. B. Styrol-Butadien, Styrol-Acrylnitril, Styrol-Ethylmethacrylat, Styrol-Butadien-Ethylacrylat, Styrol-Acrylnitril-Methacrylat;

ABS-, MBS- oder ähnliche Polymere;

Halogenhaltige Polymere, wie z. B. Polyvinylchlorid, Polyvinylfluorid, Polyvinylidenflourid sowie deren Copolymere;
Polymere die sich von $\alpha$, $\beta$-ungesättigten Säuren und deren Derivaten ableiten, wie Polyacrylate, Polymethacrylate, Polyacrylamide und Polyacrylnitrile;
Polymere, die sich von ungesättigten Alkoholen und Aminen bzw. von deren Acrylderivaten oder Acetalen ableiten, z.B. Polyvinylalkohol und Polyvinylacetat;
Polyurethane, Polyamide, Polyharnstoffe, Polyester, Polycarbonate, Polysulfone, Polyethersulfone und Polyetherketone.

Weiterhin können mit den erfindungsgemäßen Verbindungen Lacküberzüge stabilisiert werden, z.B. Industrielackierungen. Unter diesen sind Einbrennlackierungen, unter diesen wiederum Fahrzeuglackierungen, vorzugsweise Zweischichtlackierungen, besonders hervorzuheben.

Auch hier können zusätzlich die bereits genannten Antioxidatien und Lichtschutzmittel mitverwendet werden.

Die erfindungsgemäßen Verbindungen können in fester oder gelöster Form dem Lack zugesetzt werden. Ihre gute Löslichkeit in Lacksystemen ist dabei von besonderem Vorteil.

Bevorzugt werden die erfindungsgemäßen Verbindungen zum Stabilisieren von Polyolefinen, vorzugsweise von Ethylen- oder Propylenpolymerisaten, sowie von Polyurethanen verwendet.

Die Erfindung wird durch die nachstehenden Beispiele weiter erläutert.

Beispiel 1

78 g 2,2,6,6-Tetramethyl-4-aminopiperidin wurden in 250 ml Ameisensäureethylester 10,5 h am Rückfluß gekocht. Der ausgefallene Niederschlag wurde abgesaugt, mit etwas Petrolether gewaschen und aus Toluol umkristallisiert. Man isolierte 4-(Formylamino)-2,2,6,6-tetramethylpiperidin als farblosen Feststoff vom Schmelzpunkt 153 °C.

Beispiel 2

56 g 4-(N-Butylamino-2,2,6,6-tetramethylpiperidin und 25 g Tetrabutylorthotitanat wurden in 135 ml Ameisensäureethylester 4 h am Rückfluß gekocht.

Die Reaktionsmischung wurde destilliert und erhielt 4-(N-Butyl-N-formylamino)-2,2,6,6-tetramethylpiperidin als farbloses Öl von Siedepunkt 117 bis 118 °C/0,7 mbar (0,5 torr).

Beispiel 3

93 g 4-(N-Octylamino-2,2,6,6-tetramethylpiperidin und 33 g Tetrabutylorthotitanat wurden in 175 ml Ameisensäureethylester 10,5 h am Rückfluß gekocht. Aufarbeitung wie in Beispiel 2 ergab 4-(N-Octyl-N-formylamino)-2,2,6,6-tetramethylpiperidin als farbloses Öl vom Siedepunkt 145 bis 147 °C/0,7 mbar (0,5 torr).

Beispiel 4

81 g 4-(N-Cyclopentylamino)-2,2,6,6-tetramethylpiperidin und 34 g Tetrabutylorthotitanat wurden in 180 ml Ameisensäureethylester 14,5 h am Rückfluß gekocht. Nach Abdestillieren der flüchtigen Bestandteile bei 70 °C und 1,3 mbar (1 torr) wurde der kristalline Rückstand aus Petrolether umkristallisiert. Das farblose 4-(N-Cyclopentyl-N-formylamino)-2,2,6,6-tetramethylpiperidinschmilzt bei 95 °C.

Beispiel 5

80 g 4-(N-Cyclohexylamino)-2,2,6,6-tetramethylpiperidin und 32 g Tetrabutylorthotitanat wurden in 175 ml Ameisensäureethylester 11 h am Rückfluß gekocht. Nach fraktionierender Destillation erhält man bei 0,5 mbar (0,4 torr) eine Fraktion vom Siedepunkt 143 - 144 °C. Diese wurde nach dem Erstarren aus n-Hexan umkristallisiert. Man isolierte 4-(N-Cyclohexyl-N-formylamino)-2,2,6,6-tetramethylpiperidin als farblosen Feststoff vom Schmelzpunkt 105 °C.

Beispiel 6

114 g 4-(N-Phenylethylamino)-2,2,6,6-tetramethylpiperidin und 41,3 g Tetrabutylorthotitanat wurden in 220 ml Ameisensäureethylester 14,5 h am Rückfluß gekocht. Nach Abdestillieren der flüchtigen Bestandteile bei 140 °C/1,3 mbar (1 torr) wurde der erstarrte Rückstand aus Petrolether umkristallisiert. Man isoliert 4-(N-Phenylethyl-N-formylamino)-2,2,6,6-tetramethylpiperidinvom Schmelzpunkt 86 °C.

Beispiel 7

106,5 g 4-[N-(2,2,6,6-Tetramethyl-4-piperidinylamino)]-2,2,6,6-tetramethylpiperidin und 68 g Tetrabutylorthotitanat wurden in 180 ml Ameisensäureethylester 30 h am Rückfluß gekocht. Der ausgefallene Niederschlag wurde abgesaugt und aus Methylcyclohexan umkristallisiert. Man erhält 4-[N-(2,2,6,6-Tetramethyl-4-piperidinyl)-N-formylamino]-2,2,6,6-tetramethylpiperidin vom Schmelzpunkt 188 °C.

Beispiel 8

10,8 g N,N'-Bis-[2,2,6,6-Tetramethyl-4-piperidinyl]-ethylendiamin und 6 g Tetrabutylorthotitanat wurden in 70 ml Ameisensäureethylester 10,5 h am Rückfluß gekocht. Der ausgefallene Niederschlag wurde abgesaugt. Umkristallisation aus Toluol lieferte N,N'-Bis-[2,2,6,6-tetramethyl-4-piperidinyl]-N,N'-bis-formyl-ethylendiamin (III) vom Schmelzpunkt 210 °C.

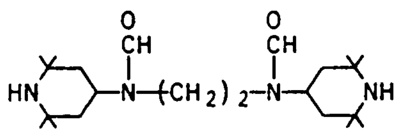

(III)

Beispiel 9

37 g N,N'-Bis-[2,2,6,6-tetramethyl-4-piperidinyl]-hexamethylendiamin und 3,2 g Tetrabutylorthotitanat wurden in 47 ml Ameisensäureethylester 36 h am Rückfluß gekocht. Der ausgefallene Niederschlag wurde abgesaugt. umkristallisation aus Essigsäureethylester lieferte N,N'-Bis-[2,2,6,6-tetramethyl-4-piperidinyl]-N,N'-bis-formyl-hexamethylendiamin (IV) vom Schmelzpunkt 155 °C.

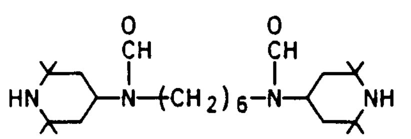

(IV)

Beispiel 10

a) 155 g 2,2,6,6-Tetramethyl-4-piperidinon, 37 g 1,3-Diaminopropan und 16 g Lewatit® S 100 wurden in 400 ml iso-Butanol am Wasserabscheider gekocht, bis sich kein Wasser mehr abschied. Der Katalysator wurde abfiltriert, die Lösung mit 40 g Natriumborhydrid versetzt und 4 Stunden auf 50 °C erwärmt. Nach

Ausschütteln mit Wasser wurde das Lösungsmittel im Vakuum entfernt und der Rückstand fraktionierend destilliert.
Ausbeute: 64,4 g der Verbindung der Formel

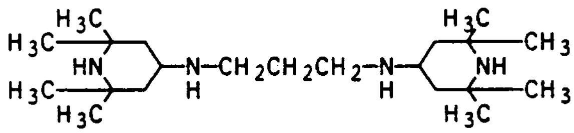

farbloses Öl; Siedepunkt 150 bis 164° C bei 0,3 mbar (0,2 torr).
b) 14,6 g Ameisensäure und 32,1 g Acetanhydrid wurden vermischt und 20 min. gerührt. Nach Zugabe von 100 ml Toluol wurde eine Lösung von 28 g des Produkts aus Beispiel a) in 70 ml Toluol zugetropft. Nach 16 Stunden bei Raumtemperatur wurden 150 ml Wasser zugegeben und mit Natronlauge alkalisch gestellt. Die Toluolphase wurde abgetrennt, die wäßrige Phase mit n-Butanol extrahiert, die organischen Phasen vereinigt und die Lösungsmittel im Wasserstrahlvakuum entfernt. Der Rückstand wurde aus Methylcyclohexan umkristallisiert.
Ausbeute: 20 g der Verbindung der Formel

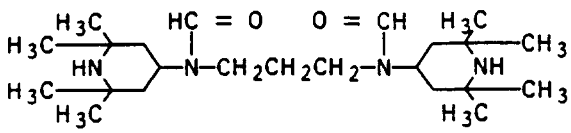

farbloser Feststoff; Schmelzpunkt 128 bis 129° C.

| | | | | |
|---|---|---|---|---|
| Ber.: | C 67,6 | H 10,8 | N 13,7 | O 7,8 % |
| Gef.: | C 67,0 | H 10,8 | N 13,4 | O 8,6 % |

Beispiel 11

a) 70,2 g 2,2,6,6-Tetramethyl-4-aminopiperidin, 27,8 g 1,4-Bis-brommethyl-benzol und 1 g Kaliumjodid wurden 0,5 Stunden auf 90° C erhitzt. Nach Zugabe von 250 ml Acetonitril wurde 2 Stunden bei Raumtemperatur gerührt. Der ausgefallene Niederschlag wurde abgesaugt, in Wasser verrührt und mit Natronlauge alkalisch gestellt. Mit n-Butanol wurde ausgeschüttelt, das Lösungsmittel im Wasserstrahlvakuum entfernt. Der Rückstand wurde in Acetonitril zum Sieden erhitzt und der ausgefallene Niederschlag bei Raumtemperatur abgesaugt.

Man erhielt 19 g der Verbindung der Formel

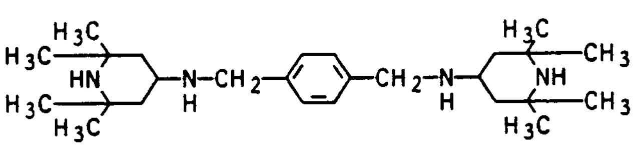

als farblosen Feststoff vom Schmelzpunkt 99 bis 100° C.

| | | | |
|---|---|---|---|
| Ber.: | C 75,3 | H 11,2 | N 13,5 % |
| Gef.: | C 72,8 | H 10,8 | N 12,8 % |

b) 8,1 g Ameisensäure, 17,8 g Acetanhydrid und 18,3 g des Produkts aus a) wurden wie in Beispiel 10b) umgesetzt und aufgearbeitet. Nach umkristallisation aus Acetonitril erhielt man 10 g der Verbindung der Formel

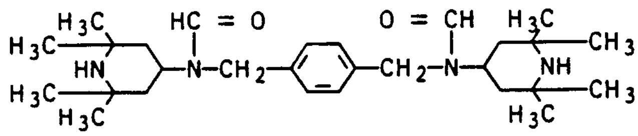

als farblosen Feststoff vom Schmelzpunkt 188 ° C.

| | | | | |
|---|---|---|---|---|
| Ber.: | C 71,4 | H 9,8 | N 11,9 | O 6,8 % |
| Gef.: | C 71,5 | H 9,9 | N 11,8 | O 6,9 % |

Beispiel 12

a) 155 g 2,2,6,6-Tetramethylpiperidon und 87,2 g 1-Pentylamin wurden wie in Beispiel 10a) umgesetzt und aufgearbeitet. Nach Destillation im Ölpumpenvakuum erhielt man 92, 1 g der Verbindung der Formel

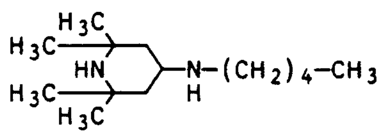

als farblose Flüssigkeit vom Siedepunkt 93 ° C bei 1,3 mber (1 torr).

b) 68 g des Produkts aus a) und 5,2 g einer 30 %igen methanolischen Natriummethylatlösung wurden in 250 ml Ameisensäuremethylester 6 Stunden zum Rückfluß erhitzt. Der überschüssige Ester wurde im Wasserstrahlvakuum entfernt, der Rückstand im Ölpumpenvakuum destilliert. Man erhielt 51 g der Verbindung der Formel

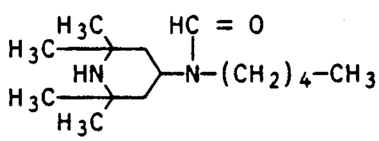

als farblose Flüssigkeit vom Siedepunkt 126 bis 127 ° C/0,4 mbar (0,3 torr).

| | | | | |
|---|---|---|---|---|
| Ber.: | C 70,8 | H 11,9 | N 11,0 | O 6,3 % |
| Gef.: | C 70,5 | H 11,8 | N 11,3 | O 6,9 % |

Beispiel 13

a) Zu 78 g 2,2,6,6-Tetramethyl-4-amino-piperidin und 1 g Kaliumjodid in 200 ml Acetonitril wurden 82,5 g 1-Bromhexan in 100 ml Acetonitril getropft. Man rührte 6 Stunden bei Raumtemperatur und 10 Stunden bei 40 bis 50 ° C. Die Mischung wurde filtriert, das Filtrat im Wasserstrahlvakuum eingeengt, der Rückstand in Wasser gelöst und unter Kühlung mit Natronlauge alkalisch gestellt. Nach dem Ausschütteln mit n-Butanol wurde das Lösungsmittel im Wasserstrahlvakuum entfernt und der Rückstand im Wasserstrahlvakuum destilliert. Man erhielt 54 g der Verbindung der Formel

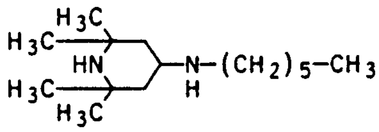

als farblose Flüssigkeit vom Siedepunkt 152 bis 154 ° C/19 mbar (14 torr).

| | | | |
|---|---|---|---|
| Ber.: | C 74,9 | H 13,4 | N 11,6 % |
| Gef.: | C 75,5 | H 13,8 | N 11,2 % |

b) 38 g des Produkts aus a) wurden wie in Beispiel 12b) mit Ameisensäuremethylester umgesetzt und aufgearbeitet. Man erhielt 24,7 g der Verbindung der Formel

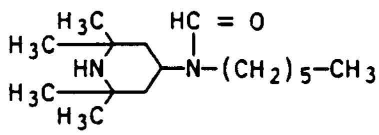

als farblose Flüssigkeit vom Siedepunkt 132 ° C/ 1,3 mbar (1 torr).

| | | | | |
|---|---|---|---|---|
| Ber.: | C 71,6 | H 12,0 | N 10,4 | O 5,9 % |
| Gef.: | C 72,3 | H 12,2 | N 10,5 | O 5,6 % |

Beispiel 14

a) 156 g Decanal und 156 g 2,2,6,6-Tetramethyl-4-amino-piperidin wurden in 500 ml Toluol am Wasserabscheider erhitzt, bis sich kein Wasser mehr abschied. Das Toluol wurde im Wasserstrahlvakuum entfernt, der Rückstand mit 600 ml Methanol und 40 g Natriumborhydrid versetzt und 4 Stunden auf 50 ° C erhitzt. Das Methanol wurde abdestilliert und der Rückstand mit Essigsäureethylester und Wasser ausgeschüttelt. Nach Phasentrennung und Trocknen der Essigesterphase wurde das Lösungsmittel im Wasserstrahlvakuum entfernt und der Rückstand im Ölpumpenvakuum destilliert. Man erhielt 46,3 g der Verbindung der Formel

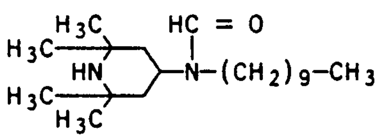

als gelbliche Flüssigkeit vom Siedepunkt 144 bis 146 ° C/0,13 mbar (0,1 torr).

b) 13,8 g Ameisensäure und 30 g Acetanhydrid wurden 0,5 Stunden verrührt. Nach Zugabe von 150 ml Dichlormethan wurden 45 g des Produktes aus a) in 100 ml Dichlormethan zugetropft. Nach 5 Stunden Rühren bei Raumtemperatur wurden 250 ml Eiswasser zugegeben, mit Natronlauge wurde alkalisch gestellt und nach Phasentrennung die organische Phase im Waserstrahlvakuum destilliert. Man erhielt 25 g der Verbindung der Formel

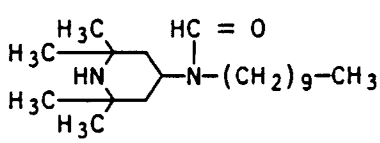

als farblose Flüssigkeit vom Siedepunkt 176 bis 178 ° C/0,13 mbar (0,1 torr).

| | | | | |
|---|---|---|---|---|
| Ber.: | C 74,0 | H 12,4 | N 8,6 | O 4,9 % |
| Gef.: | C 73,8 | H 12,4 | N 8,8 | O 5,4 % |

Beispiel 15

a) 155 g 2,2,6,6-Tetramethyl-4-piperidon und 185 g 1-Aminododecan wurden wie in Beispiel 10a) umgesetzt und aufgearbeitet. Man erhielt 84,9 g der Verbindung der Formel

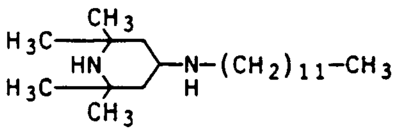

als gelbliche Flüssigkeit vom Siedepunkt 120 bis 126 ° C.

b) 35,6 g des Produkts aus a) wurden wie in Beispiel 12b) umgesetzt. Die flüchtigen Bestandteile wurden bis zu einer Temperatur von 190 ° C bei einem Druck von 0,5 mbar (0,4 torr) abdestilliert, der Rückstand bestand aus 33,3 g der Verbindung der Formel

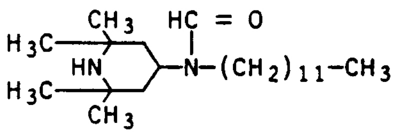

| | | | | |
|---|---|---|---|---|
| Ber.: | C 74,9 | H 12,6 | N 7,9 | O 4,5 % |
| Gef.: | C 75,4 | H 12,9 | N 5,8 | O 6,1 % |

Beispiel 16

a) 155 g 2,2,6,6-Tetramethyl-4-piperidon und 269,5 g 1-Aminooctadecan wurden wie in Beispiel 10a) umgesetzt und aufgearbeitet.

Man erhielt 54,2 g der Verbindung der Formel

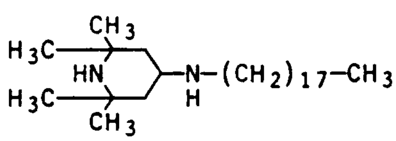

als farbloses Öl vom Siedepunkt 176 bis 180 ° C/0,4 mbar (0,3 torr).

b) 40 g des Produktes aus a) wurden wie in Beispiel 12b) umgesetzt und aufgearbeitet. Der nach dem Entfernen des Essigsäureethylesters verbliebene Rückstand wurde aus Acetonitril umkristallisiert. Man erhielt 19 g der Verbindung der Formel

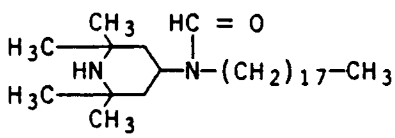

als farblosen Feststoff vom Schmelzpunkt 60 ° C.

| | | | | |
|---|---|---|---|---|
| Ber.: | C 77,0 | H 12,9 | N 6,4 | O 3,7 % |
| Gef.: | C 76,7 | H 12,9 | N 6,3 | O 3,9 % |

Beispiel 17

a) 156 g 2,2,6,6-Tetramethyl-4-amino-piperidin, 87 g Aceton und 5 g p-Toluolsulfonsäuremonohydrat wurden in 300 ml Toluol am Wasserabscheider gekocht. Nach 4,5 Stunden wurden weitere 29 g Aceton, nach 7 Stunden 29 g Aceton, nach 11 Stunden 87 g Aceton und 5 g p-Toluolsulfonsäuremonohydrat, nach 13,5 Stunden 30 g Aceton und nach 20 Stunden nochmals 30 g Aceton zugegeben. Nach weiteren 2 Stunden wurde das Lösungsmittel im Wasserstrahlvakuum entfernt, der Rückstand mit 250 ml Methanol und 38 g Natriumborhydrid versetzt und 2 Stunden gekocht. Anschließend wurde mit 400 ml Wasser verdünnt und mit Dichlormethan extrahiert. Nach Phasentrennung und Trocknen der organischen Phase über Magnesiumsulfat wurde das Lösungsmittel im Wasserstrahlvakuum entfernt und der Rückstand fraktionierend destilliert. Man erhielt 58 g der Verbindung der Formel

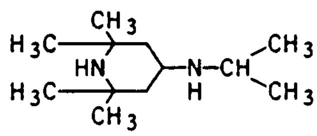

als farblose Flüssigkeit vom Siedepunkt 100 bis 102 ° C/32 mbar (24 torr).

b) 55 g des Produktes aus a) wurden wie in Beispiel 14b) umgesetzt und aufgearbeitet. Nach dem Einengen der organischen Phase wurde der Rückstand aus Acetonitril umkristallisiert. Man erhielt 21 g der Verbindung der Formel

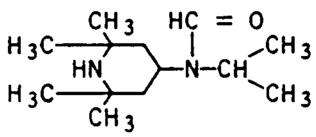

als farblosen Feststoff vom Schmelzpunkt 142 ° C.

| | | | | |
|---|---|---|---|---|
| Ber.: | C 69,0 | H 11,6 | N 12,4 | O 7,1 % |
| Gef.: | C 68,8 | H 11,5 | N 12,5 | O 6,6 % |

Beispiel 18

a) 108 g Isobutyraldehyd und 257 g 2,2,6,6-Tetramethyl-4-amino-piperidin wurden wie in Beispiel 14a) umgesetzt und aufgearbeitet. Man erhielt 218 g der Verbindung der Formel

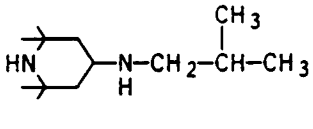

als farblose Flüssigkeit vom Siedepunkt 122 ° C/37 mbar (28 torr).

| | | | |
|---|---|---|---|
| Ber.: | C 73,5 | H 13,3 | N 13,2 % |
| Gef.: | C 73,2 | H 13,1 | N 13,4 % |

b) 63,6 g des Produkts aus a) wurden wie in Beispiel 14b) umgesetzt und aufgearbeitet. Man erhielt 50 g der Verbindung der Formel

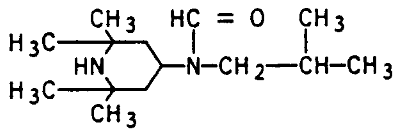

als farbloses Öl vom Siedepunkt 136° C/3,3 mbar (2,5 torr).

Nach Stehenlassen erstarrt das Öl zu einem farblosen Feststoff vom Schmelzpunkt 36° C.

| | | | | |
|---|---|---|---|---|
| Ber.: | C 69,9 | H 11,7 | N 11,6 | O 6,6 % |
| Gef.: | C 69,8 | H 11,4 | N 11,9 | O 7,5 % |

Beispiel 19

a) Zu einer Mischung von 156 g 2,2,6,6-Tetramethyl-4-aminopiperidin, 101 g Triethylamin, 1 g Kaliumiodid und 150 ml Acetonitril wurden 151 g 1-Brom-3-methylbutan in 100 ml Acetonitril getropft, 5 Stunden bei Raumtemperatur und 4 Stunden am Rückfluß gerührt.

Das Lösungsmittel wurde am Wasserstrahlvakuum entfernt, der Rückstand in Wasser gelöst und mit Natronlauge alkalisch gestellt. Nach Ausschütteln mit n-Butanol und Phasentrennung wurde die organische Phase im Wasserstrahlvakuum fraktionierend destilliert. Man erhielt 95 g der Verbindung der Formel

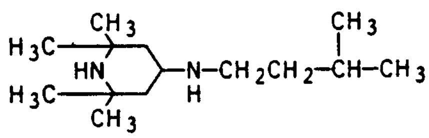

als farblose Flüssigkeit vom Siedepunkt 125 bis 126° C/16 mbar (12 totr).

| | | | |
|---|---|---|---|
| Ber.: | C 74,3 | H 13,3 | N 12,4 % |
| Gef.: | C 74,1 | H 13,2 | N 12,6 % |

b) 53 g des Produkts aus a) wurden wie in Beispiel 12b) umgesetzt und aufgearbeitet. Man erhielt 45 g der Verbindung der Formel

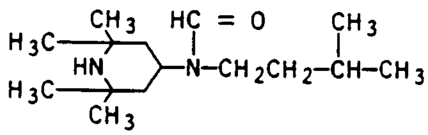

als farbloses Öl vom Siedepunkt 120 bis 121° C/0,4 mbar (0,3 torr).

| | | | | |
|---|---|---|---|---|
| Ber.: | C 70,8 | H 11,9 | N 11,0 | O 6,3 % |
| Gef.: | C 70,8 | H 11,5 | N 10,9 | O 6,5 % |

Beispiel 20

a) 125 g 3,3-Dimethyl-2-butanon, 203 g 2,2,6,6-Tetramethyl-4-aminopiperidin und 5 g p-Toluolsulfonsäuremonohydrat wurden am Wasserabscheider gekocht, bis sich kein Wasser mehr abschied. Das Lösungsmittel wurde im Wasserstrahlvakuum entfernt, der Rückstand mit 300 ml Methanol und 47,5 g Natriumborhydrid versetzt und 4 Stunden zum Rückfluß erhitzt. Man verdünnte mit 400 ml Eiswasser, extrahierte mit Dichlormethan und engte nach der Phasentrennung die organische Phase ein. Der Rückstand wurde fraktionierend destilliert. Man erhielt 164 g der Verbindung der Formel

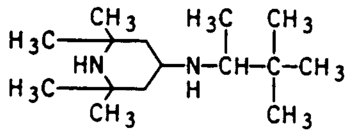

als farblose Flüssigkeit vom Siedepunkt 84 bis 86 ° C/0,7 mbar (0,5 torr).

| | | | |
|---|---|---|---|
| Ber.: | C 74,9 | H 13,4 | N 11,6 % |
| Gef.: | C 74,3 | H 13,2 | N 12,5 % |

b) 60 g des Produkts aus a) wurden wie in Beispiel 14b) umgesetzt und aufgearbeitet. Man erhielt 44 g der Verbindung der Formel

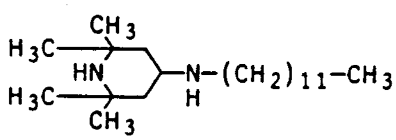

als farblose Flüssigkeit vom Siedepunkt 123 ° C/0,4 mbar (0,3 torr).

| | | | | |
|---|---|---|---|---|
| Ber.: | C 71,6 | H 12,0 | N 10,4 | O 6,0 % |
| Gef.: | C 71,5 | H 11,8 | N 10,6 | O 6,7 % |

Beispiel 21

a) 125 g 4-Methyl-2-pentanon, 202 g 2,2,6,6-Tetramethyl-4-aminopiperidin und 5 g p-Toluolsulfonsäuremonohydrat wurden wie in Beispiel 20a) umgesetzt und aufgearbeitet. Man erhielt 203 g der Verbindung der Formel

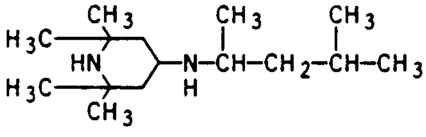

als farblose Flüssigkeit vom Siedepunkt 130 bis 131 ° C/28 mbar (21 torr).

| | | | |
|---|---|---|---|
| Ber.: | C 74,9 | H 13,4 | N 11,7 % |
| Gef.: | C 74,5 | H 13,4 | N 12,1 % |

b) 60 g des Produkts aus a) wurden wie in Beispiel 14b) umgesetzt und aufgearbeitet. Man erhielt 45 g

der Verbindung der Formel

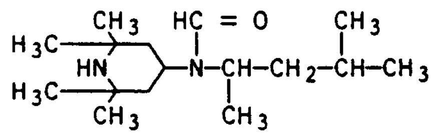

als farblose Flüssigkeit vom Siedepunkt 120 ° C/0,4 mbar (0,3 torr).

| | | | | |
|---|---|---|---|---|
| Ber.: | C 71,6 | H 12,0 | N 10,4 | O 6,0 % |
| Gef.: | C 70,9 | H 11,9 | N 10,8 | O 6,7 % |

Beispiel 22

a) Zu 78 g 2,2,6,6-Tetramethyl-4-aminopiperidin und 1 g Kaliumjodid in 200 ml Acetonitril wurden 96,5 g 1-Brom-2-ethyl-hexan in 100 ml Acetonitril zugetropft. Nach 4,5 Stunden bei Rühren bei Raumtemperatur und 8 Stunden bei 50 ° C wurden weitere 78 g 2,2,6,6-Tetramethyl-4-amino-piperidin zugetropft und die Mischung unter Rühren 2 Stunden auf 50 ° C gehalten.

Nach dem Abkühlen wurde filtriert, das Filtrat wurde vom Lösungsmittel befreit und der so erhaltene Rückstand in Wasser gelöst und mit Natronlauge alkalisch gestellt. Nach dem Ausschütteln mit n-Butanol und Phasentrennung wurde das n-Butanol im Wasserstrahlvakuum entfernt und der Rückstand im Ölpumpenvakuum fraktionierend destilliert. Man erhielt 51,3 g der Verbindung der Formel

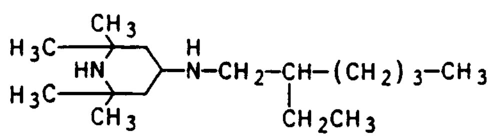

als farblose Flüssigkeit vom Siedepunkt 124 bis 126 ° C/2 mbar (1,5 torr).

| | | | |
|---|---|---|---|
| Ber.: | C 76,0 | H 13,5 | N 10,4 % |
| Gef.: | C 75,5 | H 13,3 | N 10,6 % |

b) 37 g des Produkts aus a) wurden wie in Beispiel 12b) umgesetzt (Reaktionsdauer 11 Stunden) und aufgearbeitet. Man erhielt 26 g der Verbindung der Formel

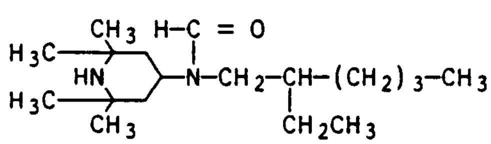

als farblose Flüssigkeit vom Siedepunkt 156 ° C/1,3 mbar (1 torr).

| | | | | |
|---|---|---|---|---|
| Ber.: | C 72,9 | H 12,2 | N 9,4 | O 5,4 % |
| Gef.: | C 72,9 | H 12,0 | N 9,8 | O 5,6 % |

Beispiel 23

a) 89 g 4-Methyl-cyclohexanon, 129 g 2,2,6,6-Tetramethylpiperidin und 5 g p-Toluolsulfonsäuremonohydrat wurden wie in Beispiel 20a) umgesetzt und aufgearbeitet. Man erhielt 76 g der Verbindung der Formel

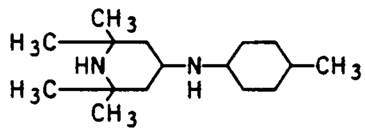

als farblose Flüssigkeit vom Siedepunkt 174 bis 176 ° C/27 mbar (20 torr).

| | | | |
|---|---|---|---|
| Ber.: | C 76,1 | H 12,8 | N 11,1 % |
| Gef.: | C 76,7 | H 12,6 | N 10,8 % |

b) 45 g des Produkts aus a) wurden wie in Beispiel 14b) umgesetzt und aufgearbeitet. Nach Entfernen des Lösungsmittels wurde der Rückstand aus n-Hexan umkristallisiert. Man erhielt 14 g der Verbindung der Formel

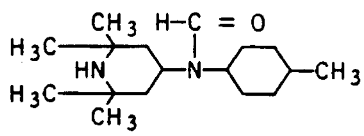

als farblosen Feststoff vom Schmelzpunkt 134 ° C.

| | | | | |
|---|---|---|---|---|
| Ber.: | C 72,8 | H 11,5 | N 10,0 | O 5,7 % |
| Gef.: | C 72,9 | H 11,6 | N 9,8 | O 5,8 % |

Beispiel 24

a) 182 g Benzophenon, 171,6 g 2,2,6,6-Tetramethyl-4-aminopiperidin und 5 g p-Toluolsulfonsäuremonohydrat wurden in 350 ml Xylol am Wasserabscheider gekocht, bis sich kein Wasser mehr abschied. Nach dem Abkühlen wurde eingeengt, mit 300 ml Methanol und 19 g Natriumborhydrid versetzt. Nach 2,5 Stunden Rückfluß wurden weitere 19 g Natriumborhydrid eingetragen und weitere 5 Stunden gekocht. Das Gemisch wurde in 2 l Wasser ausgetragen, der ausgefallene Niederschlag abgesaugt und aus iso-Propanol umkristallisiert. Man erhielt 133 g der Verbindung der Formel

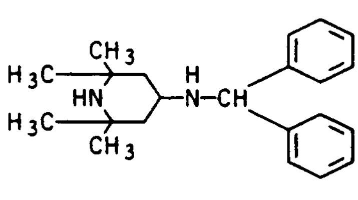

als farblosen Feststoff vom Schmelzpunkt 86 ° C.

| | | | |
|---|---|---|---|
| Ber.: | C 81,9 | H 9,4 | N 8,7 % |
| Gef.: | C 81,8 | H 9,5 | N 8,7 % |

b) 50 g des Produkts aus a) wurden wie in Beispiel 14b) umgesetzt und aufgearbeitet. Nach Entfernen des Lösungsmittels wurde der Rückstand aus n-Hexan umkristallisiert. Man erhielt 32,7 g der Verbindung

der Formel

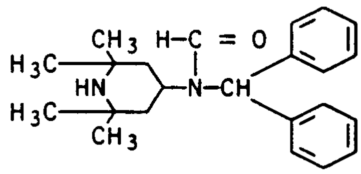

als farblosen Feststoff vom Schmelzpunkt 86° C.

| | | | | |
|---|---|---|---|---|
| Ber.: | C 78,8 | H 8,6 | N 8,0 | O 4,6 % |
| Gef.: | C 77,8 | H 8,6 | N 8,0 | O 5,0 % |

Beispiel 25

a) 126 g 1,3-Diphenylaceton, 103 g 2,2,6,6-Tetramethyl-4-amino-piperidin und 5 g p-Toluolsulfonsäuremonohydrat wurden in 350 ml Xylol am Wasserabscheider gekocht, bis sich kein Wasser mehr abschied. Nach dem Abdestillieren des Xylols wurde der Rückstand in 250 ml Methanol aufgenommen, mit 23 g Natriumborhydrid versetzt und 4 Stunden zum Rückfluß erhitzt.

Nach Zugabe von 400 ml Wasser wurde mit Dichlormethan extrahiert, nach der Phasentrennung das organische Lösungsmittel im Wasserstrahlvakuum entfernt und der so erhaltene Rückstand im Ölpumpenvakuum destilliert. Man erhielt 147 g der Verbindung der Formel

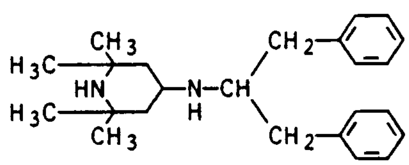

als farbloses Öl vom Siedepunkt 179° C/0,13 mbar (0,1 torr).

| | | | |
|---|---|---|---|
| Ber.: | C 82,2 | H 9,8 | N 8,0 % |
| Gef.: | C 82,1 | H 9,9 | N 8,0 % |

b) 52,5 g des Produkts aus a) wurden wie in Beispiel 14b) umgesetzt und aufgearbeitet. Nach Entfernen des Lösungsmittels wurde der erhaltene Rückstand aus Acetonitril umkristallisiert. Man erhielt 43 g der Verbindung der Formel

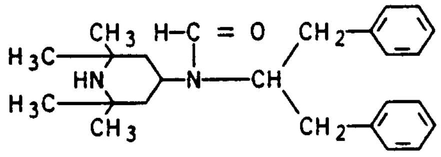

als farblosen Feststoff vom Schmelzpunkt 158° C.

| | | | | |
|---|---|---|---|---|
| Ber.: | C 79,3 | H 9,0 | N 7,4 | O 4,2 % |
| Gef.: | C 79,2 | H 9,1 | N 7,4 | O 4,4 % |

Beispiel 26

a) 78,6 g Benzylmethylketon und 91,4 g 2,2,6,6-Tetramethyl-4-aminopiperidin wurden analog Beispiel 10a) umgesetzt und aufgearbeitet. Man erhielt 43,7 g der Verbindung der Formel

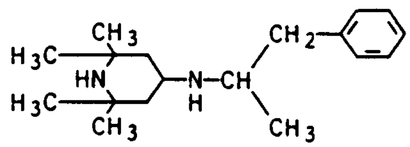

als farbloses Öl vom Siedepunkt 125 bis 127 ° C/0,4 mbar (0,3 torr).

| | | | |
|---|---|---|---|
| Ber.: | C 78,8 | H 11,0 | N 10,2 % |
| Gef.: | C 78,4 | H 10,9 | N 10,5 % |

b) 24 g des Produkts aus a) wurden wie in Beispiel 14b) umgesetzt und aufgearbeitet. Nach Entfernen des Lösungsmittels wurde der erhaltene Rückstand aus Methyl-tertiär-butylether umkristallisiert. Man erhielt 9 g der Verbindung der Formel

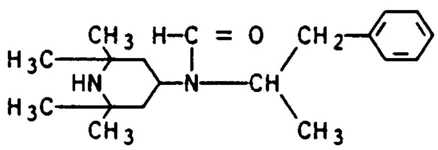

als farblosen Feststoff vom Schmelzpunkt 102 ° C.

| | | | | |
|---|---|---|---|---|
| Ber.: | C 75,4 | H 10,0 | N 9,3 | O 5,3 % |
| Gef.: | C 75,4 | H 10,0 | N 9,2 | O 5,3 % |

Beispiel 27

a) 107 g Benzylamin und 155 g 2,2,6,6-Tetramethyl-4-piperidon wurden mit 16 g Lewatit® S 100 in 400 ml Toluol am Wasserabscheider gekocht, bis sich kein Wasser mehr abschied. Die weitere Umsetzung und Aufarbeitung erfolgte wie in Beispiel 10a). Man erhielt 119 g der Verbindung der Formel

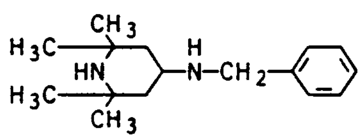

als farblose Flüssigkeit vom Siedepunkt 124 bis 126 ° C/0,7 mbar (0,5 torr).

b) 50 g des Produkts aus a) wurden analog Beispiel 12b) umgesetzt und aufgearbeitet. Nach Entfernen des Essigesters wurde der Rückstand aus Methylcyclohexan umkristallisiert. Man erhielt 41 g der Verbindung der Formel

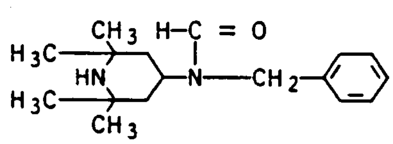

als farblosen Feststoff vom Schmelzpunkt 106 ° C.

| | | | | |
|---|---|---|---|---|
| Ber.: | C 72,0 | H 9,6 | N 9,9 | O 8,5 % |
| Gef.: | C 72,3 | H 9,2 | N 9,7 | O 8,2 % |

Die Verbindung kristallisierte mit 0,5 Mol Kristallwasser.

Beispiel 28

a) 102 g 4-Methylbenzaldehyd und 140,4 g 2,2,6,6-Tetramethyl-4-aminopiperidinwurden ohne Katalysator analog Beispiel 25a) in Xylol umgesetzt und aufgearbeitet. Nach Destillation erhielt man 170 g der Verbindung der Formel

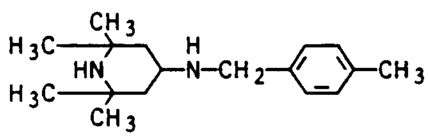

als farblose Flüssigkeit vom Siedepunkt 138° C/0,7 mbar (0,5 torr).

| | | | |
|---|---|---|---|
| Ber.: | C 78,4 | H 10,8 | N 10,7 % |
| Gef.: | C 78,1 | H 10,9 | N 11,2 % |

b) 52 g des Produkts aus a) wurden wie in Beispiel 14b) umgesetzt und aufgearbeitet. Nach Entfernen des Lösungsmittels wurde aus Acetonitril umkristallisiert. Man erhielt 39 g der Verbindung der Formel

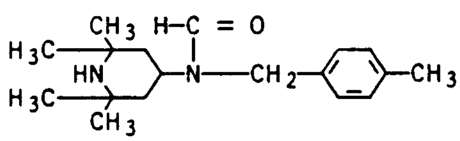

als farblosen Feststoff vom Schmelzpunkt 119° C.

| | | | | |
|---|---|---|---|---|
| Ber.: | C 75,0 | H 9,8 | N 9,7 | O 5,5 % |
| Gef.: | C 75,1 | H 9,8 | N 9,7 | O 5,6 % |

Beispiel 29

a) 136 g 4-Methoxybenzaldehyd und 172 g 2,2,6,6-Tetramethyl-4-aminopiperidin wurden wie in Beispiel 28a) umgesetzt und aufgearbeitet. Man erhielt 190 g der Verbindung der Formel

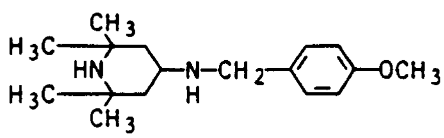

als farblose Flüssigkeit vom Siedepunkt 150 bis 152° C/0,4 mbar (0,3 torr).

| | | | | |
|---|---|---|---|---|
| Ber.: | C 73,9 | H 10,2 | N 10,1 | O 5,8 % |
| Gef.: | C 73,1 | H 10,2 | N 10,7 | O 6,2 % |

b) 55 g des Produkts aus a) wurden wie in Beispiel 14b) umgesetzt und aufgearbeitet. Nach Entfernen des Lösungsmittels wurde aus Methyltertiär-butylether umkristallisiert. Man erhielt 42 g der Verbindung der Formel

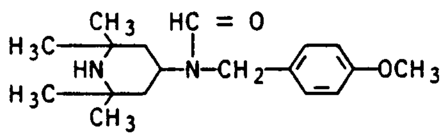

als farblosen Feststoff vom Schmelzpunkt 108 ° C.

| | | | | |
|---|---|---|---|---|
| Ber.: | C 71,0 | H 9,3 | N 9,2 | O 10,5 % |
| Gef.: | C 70,9 | H 9,4 | N 9,2 | O 10,6 % |

Beispiel 30

a) 100 g 4-Dimethylaminobenzaldehyd und 109 g 2,2,6,6-Tetramethyl-4-amino-piperidin wurden wie in Beispiel 28a) umgesetzt und aufgearbeitet. Man erhielt 142 g der Verbindung der Formel

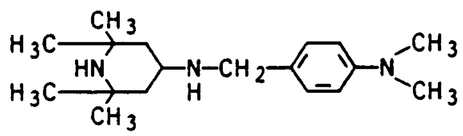

als farbloses Öl vom Siedepunkt 162 ° C/0,13 mbar (0,1 torr), das nach Stehenlassen zu einem farblosen Feststoff vom Schmelzpunkt 53 ° C erstarrt.

| | | | |
|---|---|---|---|
| Ber.: | C 74,7 | H 10,8 | N 14,5 % |
| Gef.: | C 74,7 | H 10,8 | N 14,8 % |

b) 57,8 g des Produkts aus a) wurden wie in Beispiel 14b) umgesetzt und aufgearbeitet. Nach Entfernen des Lösungsmittels wurde aus Acetonitril umkristallisiert. Man erhielt 37 g der Verbindung der Formel

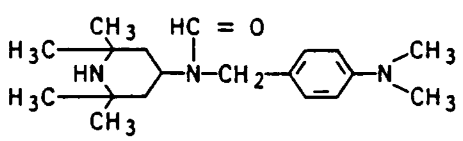

als farblosen Feststoff vom Schmelzpunkt 126 ° C.

| | | | | |
|---|---|---|---|---|
| Ber.: | C 71,9 | H 9,8 | N 13,2 | O 5,0 % |
| Gef.: | C 72,0 | H 10,0 | N 13,3 | O 5,1 % |

Beispiel 31

a) 101 g 4-Fluorbenzaldehyd und 128 g 2,2,6,6-Tetramethyl-4-aminopiperidin wurden wie in Beispiel 28a) umgesetzt und aufgearbeitet. Man erhielt 161 g der Verbindung der Formel

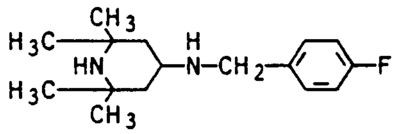

als farblose Flüssigkeit vom Siedepunkt 120 bis 122 ° C/0,3 mbar (0,2 torr).

| | | | | |
|---|---|---|---|---|
| Ber.: | C 72,7 | H 9,5 | F 7,2 | N 10,6 % |
| Gef.: | C 72,6 | H 9,6 | F 7,4 | N 10,9 % |

b) 53 g des Produkts aus a) wurden wie in Beispiel 14b) umgesetzt und aufgearbeitet. Nach Entfernen des Lösungsmittels wurde aus Acetonitril umkristallisiert. Man erhielt 25 g der Verbindung der Formel

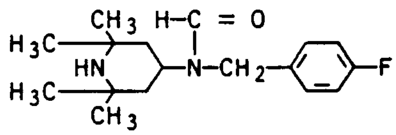

als farblosen Feststoff vom Schmelzpunkt 108 ° C.

| | | | | |
|---|---|---|---|---|
| Ber.: | C 69,8 | H 8,6 | F 6,5 | N 9,6 % |
| Gef.: | C 69,8 | H 8,7 | F 6,4 | N 9,6 % |

Beispiel 32

a) Zu 156 g 2,2,6,6-Tetramethyl-4-aminopiperidin, 101 g Triethylamin und 1 g Kaliumjodid in 100 ml Acetonitril tropfte man eine Lösung von 161 g 4-Chlorbenzylchlorid in 100 ml Acetonitril. Nach 14 Stunden Rühren bei Raumtemperatur, 1 Stunde Rückflußkochen und Abkühlen wurde der ausgefallene Niederschlag abgesaugt, mit 1 l Wasser verrührt, mit Natronlauge alkalisch gestellt, wiederum abgesaugt, mit Wasser gewaschen und aus Acetonitril umkristallisiert. Man erhielt 127 g der Verbindung der Formel

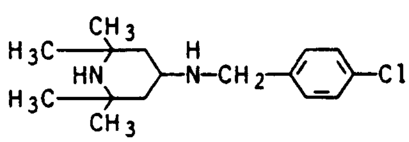

als farblosen Feststoff vom Schmelzpunkt 61 ° C.

| | | | | |
|---|---|---|---|---|
| Ber.: | C 68,4 | H 9,0 | Cl 12,6 | N 10,0 % |
| Gef.: | C 68,4 | H 9,1 | Cl 12,7 | N 9,9 % |

b) 50 g des Produkts aus a) wurden in Toluol analog Beispiel 14b) umgesetzt. Nach Entfernen des Lösungsmittels wurde aus Methyl-tertiärbutylether umkristallisiert. Man erhielt 34 g der Verbindung der Formel

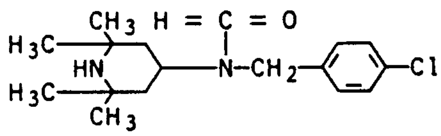

als farblosen Feststoff vom Schmelzpunkt 121 ° C.

| | | | | | |
|---|---|---|---|---|---|
| Ber.: | C 66,1 | H 8,1 | Cl 11,5 | N 9,1 | O 5,2 % |
| Gef.: | C 66,2 | H 8,3 | Cl 11,4 | N 9,1 | O 5,3 % |

Beispiel 33

a) 107 g Pyridin-3-carbaldehyd und 172 g 2,2,6,6-Tetramethyl-4-aminopiperidin wurden analog Beispiel 28a) umgesetzt und aufgearbeitet. Man erhielt 157 g der Verbindung der Formel

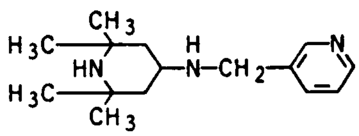

als farbloses Öl vom Siedepunkt 140 ° C/0,3 mbar (0,2 torr).

| | | | |
|---|---|---|---|
| Ber.: | C 72,8 | H 10,2 | N 17,0 % |
| Gef.: | C 72,1 | H 10,3 | N 17,0 % |

b) 49,4 g des Produkts aus a) wurden wie in Beispiel 14b) umgesetzt und aufgearbeitet. Nach Entfernen des Lösungsmittels wurde aus Acetonitril umkristallisiert. Man erhielt 28 g der Verbindung der Formel

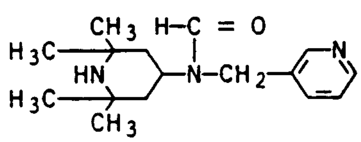

als farblosen Feststoff vom Schmelzpunkt 133 ° C.

| | | | | |
|---|---|---|---|---|
| Ber.: | C 69,8 | H 9,1 | N 15,2 | O 5,8 % |
| Gef.: | C 69,8 | H 9,2 | N 15,3 | O 6,0 % |

Beispiel 34

a) 310 g 2, 2, 6, 6-Tetramethyl-4-piperidin und 178 g 2-Ethoxy-ethylamin wurden wie in Beispiel 10a) umgesetzt und aufgearbeitet. Man erhielt 88,4 g der Verbindung der Formel

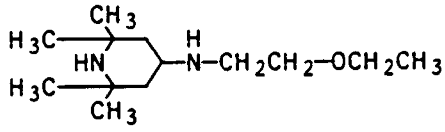

als farblose Flüssigkeit vom Siedepunkt 84 ° C/3 mbar (2 torr).

| | | | | |
|---|---|---|---|---|
| Ber.: | C 68,4 | H 12,4 | N 12,3 | O 7,0 % |
| Gef.: | C 67,3 | H 12,4 | N 12,2 | O 7,7 % |

b) 45 g des Produkts aus a) wurden wie in Beispiel 12b) umgesetzt und aufgearbeitet. Man erhielt 31,5 g der Verbindung der Formel

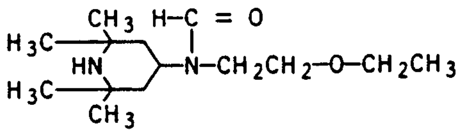

als farblose Flüssigkeit vom Siedepunkt 118 ° C/0,3 mbar (0,2 torr).

| | | | | |
|---|---|---|---|---|
| Ber.: | C 65,6 | H 11,0 | N 10,9 | O 12,5 % |
| Gef.: | C 65,2 | H 11,2 | N 11,1 | O 13,0 % |

Beispiel 35

a) 89 g 3-Ethoxy-propylamin und 155 g 2,2,6,6-Tetramethyl-4-piperidon wurden wie in Beispiel 10a) umgesetzt und aufgearbeitet. Man erhielt 61,3 g der Verbindung der Formel

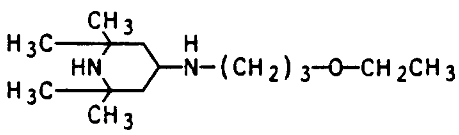

als farblose Flüssigkeit vom Siedepunkt 108 bis 110 ° C/2 mbar (1,5 torr).
b) 34,5 g des Produkts aus a) wurden wie in Beispiel 12b) umgesetzt und aufgearbeitet. Man erhielt 17 g der Verbindung der Formel

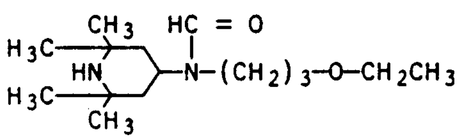

als farblose Flüssigkeit vom Siedepunkt 128 ° C/0,13 mbar (0,1 torr).

| | | | | |
|---|---|---|---|---|
| Ber.: | C 66,6 | H 11,2 | N 10,3 | O 11,8 % |
| Gef.: | C 66,2 | H 11,1 | N 10,6 | O 12,4 % |

Beispiel 36

a) 187 g 3-(2-Ethylhexoxy)propylamin und 155 g 2,2,6,6-Tetramethyl-4-piperidinon wurden wie in Beispiel 10 a umgesetzt und aufgearbeitet.
Man erhielt 191 g der Verbindung der Formel

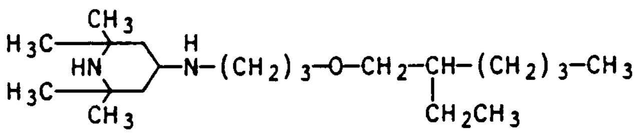

als farbloses Öl vom Siedepunkt 122 ° C/2 mbar (1,5 torr).

| | | | | |
|---|---|---|---|---|
| Ber.: | C 73,6 | H 13,0 | N 8,6 | O 4,9 % |
| Gef.: | C 73,1 | H 12,9 | N 8,8 | O 5,6 % |

b) 32,6 g des Produkts aus a) wurden wie in Beispiel 14b) umgesetzt und aufgearbeitet. Man erhielt 23 g der Verbindung der Formel

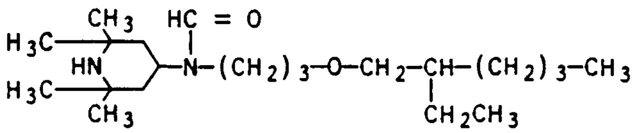

als gelbliche Flüssigkeit vom Siedepunkt 172 bis 174 ° C/0,3 mbar (0,25 torr).

| | | | | |
|---|---|---|---|---|
| Ber.: | C 71,1 | H 11,9 | N 7,9 | O 9,0 % |
| Gef.: | C 70,8 | H 11,8 | N 8,0 | O 9,9 % |

Beispiel 37

a) 102 g 3-Dimethylamino-propylamin und 155 g 2,2,6,6-Tetramethyl-4-piperidinon wurden wie in Beispiel 10a) umgesetzt und aufgearbeitet. Man erhielt 49,5 g der Verbindung der Formel

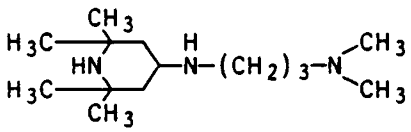

als farblose Flüssigkeit vom Siedepunkt 88 bis 92 ° C/0,2 mbar (0,15 torr).
b) 47 g des Produkts aus a) wurden wie in Beispiel 12b) umgesetzt und aufgearbeitet. Man erhielt 15 g der Verbindung der Formel

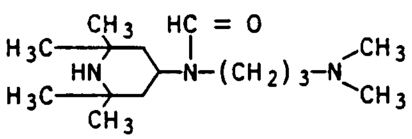

als farbloses Öl vom Siedepunkt 138 ° C/0,3 mbar (0,2 torr), das nach Stehenlassen zu einem farblosen Feststoff vom Schmelzpunkt 62 ° C erstarrt.

| | | | | |
|---|---|---|---|---|
| Ber.: | C 66,9 | H 11,6 | N 15,6 | O 5,9 % |
| Gef.: | C 67,0 | H 11,5 | N 15,4 | O 6,2 % |

Beispiel 38

a) 232 g 2-Diethylamino-ethylamin und 310 g 2,2,6,6-Tetramethyl-4-piperidinon wurden wie in Beispiel 10a) umgesetzt und aufgearbeitet. Man erhielt 185 g der Verbindung der Formel

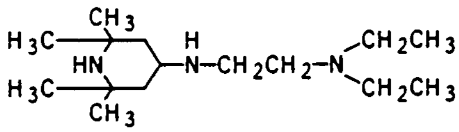

als farblose Flüssigkeit vom Siedepunkt 98 bis 100 ° C/0,4 mbar (0,3 torr).

b) 50,8 g des Produkts aus a) wurden wie in Beispiel 12b) umgesetzt und aufgearbeitet. Man erhielt 28,7 g der Verbindung der Formel

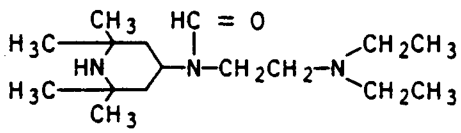

als farblose Flüssigkeit vom Siedepunkt 147 ° C/0,2 mbar (0,15 torr).

| | | | | |
|---|---|---|---|---|
| Ber.: | C 67,8 | H 11,7 | N 14,8 | O 5,7 % |
| Gef.: | C 67,2 | H 11,7 | N 15,2 | O 6,5 % |

Beispiel 39

a) 288,4 g 2-(Diisopropylamino)ethylamin und 310 g 2,2,6,6-Tetramethyl-4-piperidinon wurden wie in Beispiel 10a) umgesetzt und aufgearbeitet. Man erhielt 177,6 g der Verbindung der Formel

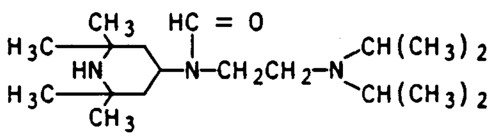

als farblose Flüssigkeit vom Siedepunkt 108 bis 110 ° C/0,5 mbar (0,35 torr).

| | | | |
|---|---|---|---|
| Ber.: | C 72,0 | H 13,2 | N 14,8 % |
| Gef.: | C 71,8 | H 13,1 | N 15,0 % |

b) 60 g des Produkts aus a) wurden wie in Beispiel 12b) umgesetzt und aufgearbeitet. Man erhielt 44,4 g der Verbindung der Formel

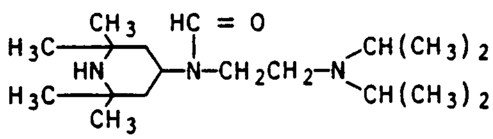

als farbloses Öl vom Siedepunkt 133 bis 134 ° C/0,13 mbar (0,1 torr), das zu einem farblosen Feststoff vom Schmelzpunkt 69 ° C erstarrt.

| | | | | |
|---|---|---|---|---|
| Ber.: | C 69,4 | H 12,0 | N 13,5 | O 5,3 % |
| Gef.: | C 69,7 | H 11,9 | N 13,7 | O 5,3 % |

Beispiel 40

a) 228,4 g 2(1-Pyrrolidyl)ethylamin und 310 g 2,2,6,6-Tetramethyl-4-piperidinon wurden wie in Beispiel 10a) umgesetzt und aufgearbeitet. Man erhielt 182 g der Verbindung der Formel

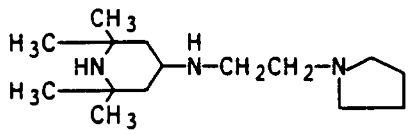

als farblose Flüssigkeit vom Siedepunkt 117 bis 121 ° C/0,13 mbar (0,1 torr).

| | | | |
|---|---|---|---|
| Ber.: | C 71,1 | H 12,3 | N 16,6 % |
| Gef.: | C 70,8 | H 12,4 | N 16,5 % |

b) 50 g des Produkts aus a) wurden wie in Beispiel 12b) umgesetzt und aufgearbeitet. Man erhielt 34 g der Verbindung der Formel

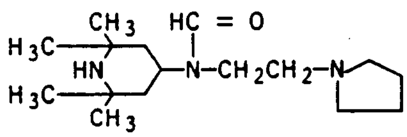

als farbloses Öl vom Siedepunkt 143 ° C/0,13 mbar (0,1 torr), das zu einem farblosen Feststoff vom Schmelzpunkt 77 ° C erstarrt.

| | | | | |
|---|---|---|---|---|
| Ber.: | C 68,3 | H 11,1 | N 14,9 | O 5,7 % |
| Gef.: | C 68,4 | H 11,3 | N 15,0 | O 5,8 % |

Beispiel 41

a) 122 g Bicyclo[2.2.1]heptan-2-on, 163,8 g 2,2,6,6-Tetramethyl-4-aminopiperidin und 5 g p-Toluolsulfonsäuremonohydrat wurden in 400 ml Xylol am Wasserabscheider erhitzt, bis sich kein Wasser mehr abschied. Nach dem Abkühlen wurde filtriert und das Filtrat im Wasserstrahlvakuum vom Lösungsmittel befreit. Nach dem Abkühlen erstarrte der Rückstand. Er bestand aus 242 g der Verbindung der Formel

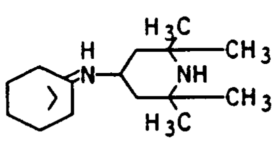

als farbloser Feststoff vom Schmelzpunkt 61 bis 62 ° C.

| | | | |
|---|---|---|---|
| Ber.: | C 77,4 | H 11,3 | N 11,3 % |
| Gef.: | C 76,9 | H 11,3 | N 11,1 % |

b) 100 g des Produkts aus a) wurden in 400 ml Toluol mit 10 g Raney-Nickel bei einem Druck von 300 bar Wasserstoff und einer Temperatur von 100 °C hydriert, bis kein Wasserstoff mehr aufgenommen wurde. Nach dem Filtrieren wurde das Lösungsmittel im Wasserstrahlvakuum entfernt, der Rückstand wurde im Ölpumpenvakuum fraktionierend destilliert. Man erhielt 71 g der Verbindung der Formel

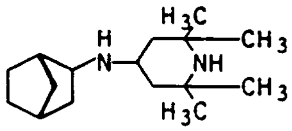

als farbloses Öl vom Siedepunkt 99 bis 100°C/0,13 mbar (0,1 torr), das zu einem farblosen Feststoff vom Schmelzpunkt 36°C erstarrt.

| | | | |
|---|---|---|---|
| Ber.: | C 76,7 | H 12,1 | N 11,2 % |
| Gef.: | C 76,7 | H 12,2 | N 11,4 % |

c) 34,5 g Ameisensäure und 76,5 g Acetanhydrid wurden 0,5 Stunden gerührt. Anschließend wurden 40 g des Produkts aus b) eingetragen. Nach Stehenlassen über Nacht wurden 450 ml Eiswasser zugegeben, mit Natronlauge wurde alkalisch gestellt und mit Dichlormethan ausgeschüttelt. Nach Trocknen über Magnesiumsulfat wurde die organische Phase vom Lösungsmittel befreit und der Rückstand aus Acetonitril umkristallisiert. Man erhielt 19 g der Verbindung der Formel

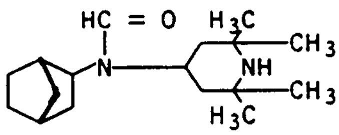

als farblosen Feststoff vom Schmelzpunkt 92°C.

| | | | | |
|---|---|---|---|---|
| Ber.: | C 73,3 | H 10,9 | N 10,1 | O 5,7 % |
| Gef.: | C 73,2 | H 10,9 | N 10,1 | O 5,8 % |

Beispiel 42

a) 118,4 g 4-Isopropylbenzaldehyd und 132,6 g 2,2,6,6-Tetramethyl-4-amino-piperidin wurden wie in Beispiel 28a) umgesetzt und aufgearbeitet. Man erhielt 165 g der Verbindung der Formel

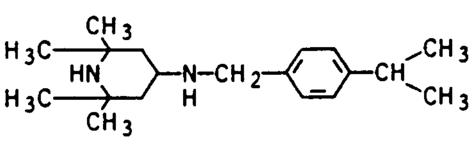

als farblose Flüssigkeit vom Siedepunkt 144 bis 145°C/0,13 mbar (0,1 torr).

| | | | |
|---|---|---|---|
| Ber.: | C 79,1 | H 11,2 | N 9,7 % |
| Gef.: | C 78,7 | H 11,2 | N 10,0 % |

b) 72 g des Produkts aus a) wurden wie in Beispiel 14b) umgesetzt und aufgearbeitet. Umkristallisation aus Acetonitril lieferte 50 g der Verbindung der Formel

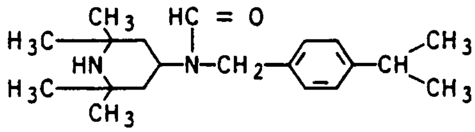

als farblosen Feststoff vom Schmelzpunkt 105 ° C.

| | | | | |
|---|---|---|---|---|
| Ber.: | C 75,9 | H 10,2 | N 8,8 | O 5,0 % |
| Gef.: | C 75,7 | H 10,2 | N 8,8 | O 5,5 % |

Beispiel 43

a) 112,5 g 3,4-(Methylendioxy)-benzaldehyd und 124 g 2,2,6,6-Tetramethyl-4-amino-piperidin wurden wie in Beispiel 28 a umgesetzt und aufgearbeitet. Man erhielt 154 g der Verbindung der Formel

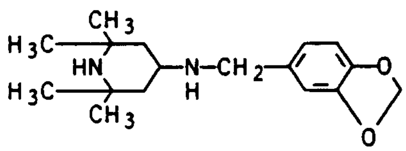

als farblose Flüssigkeit vom Siedepunkt 154 bis 155 ° C/0,13 mbar (0,1 torr).

| | | | | |
|---|---|---|---|---|
| Ber.: | C 70,3 | H 9,0 | N 9,6 | O 11,0 % |
| Gef.: | C 70,0 | H 8,9 | N 10,0 | O 11,3 % |

b) 58 g des Produkts aus a) wurden wie in Beispiel 14b) umgesetzt und aufgearbeitet. Nach Umkristallisation aus Acetonitril erhielt man 18 g der Verbindung der Formel

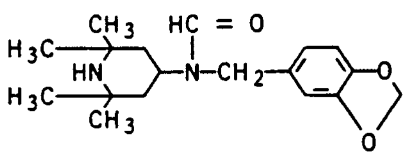

als farblosen Feststoff vom Schmelzpunkt 95 ° C.

| | | | | |
|---|---|---|---|---|
| Ber.: | C 67,9 | H 8,2 | N 8,8 | O 15,1 % |
| Gef.: | C 67,9 | H 8,4 | N 8,8 | O 14,9 % |

Beispiel 44

a) 50 g 2,4,6-Trimethylbenzaldehyd und 53 g 2,2,6,6-Tetramethyl-4-amino-piperidin wurden wie in Beispiel 28a) umgesetzt und aufgearbeitet. Man erhielt 74 g der Verbindung der Formel

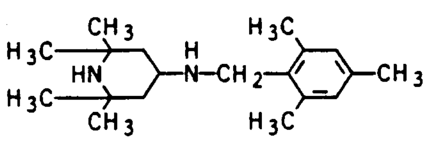

als farbloses Öl vom Siedepunkt 150 bis 151 ° C/0,13 mbar (0,1 torr), das zu einem farblosen Feststoff vom Schmelzpunkt 53 ° C erstarrt.

| | | | |
|---|---|---|---|
| Ber.: | C 79,1 | H 11,2 | N 9,7 % |
| Gef.: | C 78,6 | H 11,2 | N 9,8 % |

b) 51 g des Produkts aus a) wurden wie in Beispiel 14b) umgesetzt und aufgearbeitet. Nach Entfernen des Lösungsmittels wurde aus Acetonitril umkristallisiert. Man erhielt 42 g der Verbindung der Formel

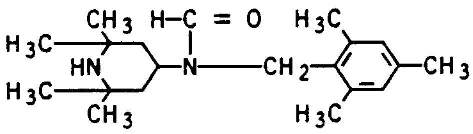

vom Schmelzpunkt 133 ° C.

| | | | | |
|---|---|---|---|---|
| Ber.: | C 75,9 | H 10,2 | N 8,8 | O 5,0 % |
| Gef.: | C 75,6 | H 10,0 | N 8,9 | O 5,1 % |

Beispiel 45

a) 68,8 g Pivalinaldehyd und 125 g 2,2,6,6-Tetramethyl-4-amino-piperidin wurden wie in Beispiel 28a) umgesetzt und aufgearbeitet. Man erhielt 115 g der Verbindung der Formel

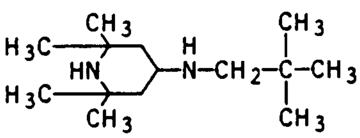

als farblose Flüssigkeit vom Siedepunkt 76 bis 77 ° C/0,4 mbar (0,3 torr).

| | | | |
|---|---|---|---|
| Ber.: | C 74,3 | H 13,3 | N 12,4 % |
| Gef.: | C 73,6 | H 13,4 | N 12,8 % |

b) 65 g der Produkts aus a) wurden wie in Beispiel 14b) umgesetzt und aufgearbeitet. Man erhielt 52 g der Verbindung der Formel

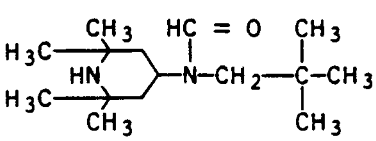

als farbloses Öl vom Siedepunkt 116 bis 118 ° C/0,13 mbar (0,1 torr), das zu einem farblosen Feststoff vom Schmelzpunkt 64 ° C erstarrt.

| | | | | |
|---|---|---|---|---|
| Ber.: | C 70,8 | H 11,9 | N 11,0 | O 6,3 % |
| Gef.: | C 70,5 | H 11,9 | N 11,0 | O 6,7 % |

Beispiel 46

a) 70 g Cyclododecanon, 61 g 2,2,6,6-Tetramethyl-4-amino-piperidin und 5 g p-Toluolsulfonsäuremonohydrat wurden wie in Beispiel 25a) umgesetzt und aufgearbeitet. Man erhielt 88 g der Verbindung der Formel

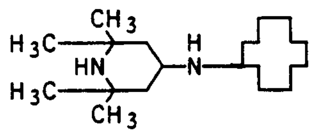

als farblose Flüssigkeit vom Siedepunkt 152 bis 154 ° C/0,2 mbar (0,15 torr).

| | | | |
|---|---|---|---|
| Ber.: | C 78,2 | H 13,1 | N 8,7 |
| Gef.: | C 78,0 | H 13,1 | N 8,8 |

b) 46 g des Produkts aus a) wurden wie in Beispiel 14b) umgesetzt und aufgearbeitet. Nach Entfernen des Lösungsmittels wurde aus Acetonitril umkristallisiert. Man erhielt 34 g der Verbindung der Formel

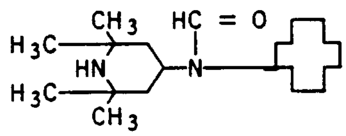

als farblosen Feststoff vom Schmelzpunkt 155 ° C.

| | | | | |
|---|---|---|---|---|
| Ber.: | C 75,4 | H 12,1 | N 8,0 | O 4,6 % |
| Gef.: | C 75,2 | H 11,9 | N 8,0 | O 4,6 % |

Beispiel 47

a) Zu 145 g 2,2,6,6-Tetramethyl-4-amino-piperidin, 94 g Triethylamin und 1 g Kaliumiodid in 150 ml Acetonitril tropfte man 112 g 4-Chlor-1-butanol in 100 ml Acetonitril, rührte 2 Stunden bei Raumtemperatur und 6,5 Stunden am Rückfluß. Der ausgefallene Niederschlag wurde abgesaugt, in Wasser gelöst, mit Natronlauge alkalisch gestellt und mit n-Butanol extrahiert. Nach Phasentrennung wurde die organische Phase im Wasserstrahlvakuum vom Lösungsmittel befreit und der Rückstand destilliert. Man erhielt 57 g der Verbindung der Formel

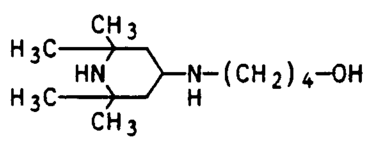

als farblose Flüssigkeit vom Siedepunkt 175 bis 177 ° C/16 mbar (12 torr), die zu einem farblosen Feststoff vom Schmelzpunkt 85 ° C erstarrt.

| | | | | |
|---|---|---|---|---|
| Ber.: | C 68,4 | H 12,3 | N 12,3 | O 7,0 % |
| Gef.: | C 68,1 | H 12,4 | N 12,1 | O 7,1 % |

b) 44,3 g des Produkts aus a) wurden wie in Beispiel 12b) umgesetzt und aufgearbeitet. Man erhielt 19,3 g eines 1 : 1-Gemisches der Verbindungen der Formeln

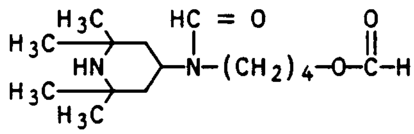 und 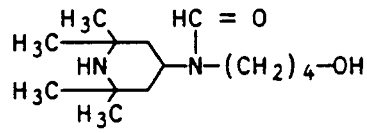

als farblose Flüssigkeit vom Siedepunkt 180 ° C/0,7 mbar (0,5 torr).

| | | | | |
|---|---|---|---|---|
| Ber.: | C 64,4 | H 10,4 | N 10,4 | O 14,8 % |
| Gef.: | C 64,6 | H 10,6 | N 10,5 | O 14,4 % |

Beispiel 48

a) 260,4 g 2-(4-Morpholinyl)ethylamin und 310 g 2,2,6,6-Tetramethyl-4-piperidon wurden wie in Beispiel 10a) umgesetzt und aufgearbeitet. Man erhielt 50 g der Verbindung der Formel

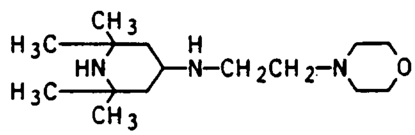

als farblose Flüssigkeit vom Siedepunkt 109 bis 111 ° C/0,4 mbar (0,3 torr).

| | | | | |
|---|---|---|---|---|
| Ber.: | C 66,8 | H 11,6 | N 15,9 | O 6,0 % |
| Gef.: | C 66,3 | H 11,6 | N 15,4 | O 6,8 % |

b) 33 g des Produkts aus a) wurden wie in Beispiel 14b) umgesetzt und aufgearbeitet. Man erhielt 29 g der Verbindung der Formel

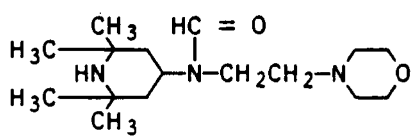

als farblose Flüssigkeit vom Siedepunkt 174 bis 176 ° C/0,7 mbar (0,5 torr), die zu einem farblosen Feststoff vom Schmelzpunkt 89 ° C erstarrt.

| | | | | |
|---|---|---|---|---|
| Ber.: | C 64,6 | H 10,5 | N 14,2 | O 10,8 % |
| Gef.: | C 64,2 | H 10,6 | N 14,8 | O 10,9 % |

Beispiel 49

80 g des Produkts aus Beispiel 5 wurden in 250 ml Essigsäureanhydrid eingetragen und das Gemisch 3,5 Stunden gekocht. Man tropfte die Reaktionsmischung in eine Mischung aus 400 g 50 %iger Natronlauge und 400 g Eis. Der ausgefallene Niederschlag wurde abgesaugt, mit Wasser neutral gewaschen, getrocknet und aus Cyclohexan umkristallisiert. Man erhielt 57 g der Verbindung der Formel

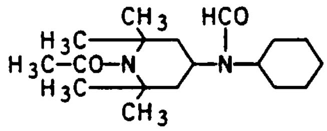

als farblosen Feststoff vom Schmp. 129 ° C.

| | | | | |
|---|---|---|---|---|
| Ber.: | C 70,1 | H 10,5 | N 9,1 | O 10,4 % |
| Gef.: | C 70,2 | H 10,5 | N 9,1 | O 10,4 % |

**Patentansprüche**

**1.** 4-Formylaminopiperidinderivate der allgemeinen Formel (I)

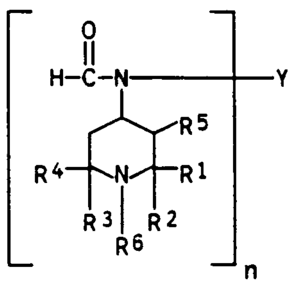

(I),

in der

| | |
|---|---|
| n | 1 oder 2, |
| $R^1$, $R^2$, $R^3$ und $R^4$ | unabhängig voneinander $C_1$- bis $C_4$-Alkyl oder |
| $R^1$ und $R^2$ oder $R^3$ und $R^4$ | zusammen eine Tetramethylen- oder Pentamethylengruppe, |
| $R^5$ | Wasserstoff oder $C_1$- bis $C_4$-Alkyl, |
| $R^6$ | Wasserstoff, $C_1$- bis $C_{22}$-Alkyl, $C_3$- bis $C_{22}$-Alkenyl, gegebenenfalls durch $C_1$-$C_4$-Alkyl, Fluor, Chlor, $C_1$-$C_4$-Alkoxy, Methylendioxy, Ethylendioxy und/oder Di-$C_1$-$C_4$-alkylamino substituiertes $C_7$- bis $C_{12}$-Phenylalkyl, $C_1$- bis $C_{22}$-Alkanoyl, $C_2$- bis $C_3$-Cyanalkyl, $C_1$- bis $C_{22}$-Hydroxyalkyl oder $C_2$- bis $C_{22}$-Aminoalkyl und |

- wenn n = 1 ist -

Y Wasserstoff, $C_1$- bis $C_{22}$-Alkyl, $C_3$- bis $C_{22}$-Alkenyl, $C_3$- bis $C_{12}$-Cycloalkyl oder Bicycloalkyl, durch Cyan, Hydroxy oder Carbo-$C_1$-$C_4$-alkoxy substituiertes $C_2$- bis $C_{22}$-Alkyl, durch Ethersauerstoff, Stickstoff oder Schwefel unterbrochenes $C_4$-$C_{22}$-Alkyl, gegebenenfalls durch $C_1$-$C_4$-Alkyl, Fluor, Chlor, $C_1$-$C_4$-Alkoxy, Methylendioxy, Ethylendioxy oder Di-$C_1$-$C_4$-alkylamino substituiertes $C_7$-bis $C_{22}$-Phenyl- oder Diphenylalkyl, gegebenenfalls durch $C_1$-$C_4$-Alkyl oder Carbo-$C_1$-$C_4$-alkoxy substituiertes Phenyl, ein Rest der Formel

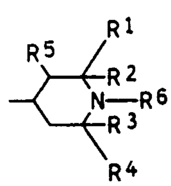

oder heterocyclische Reste der Formel

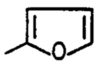 , 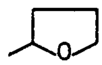 , 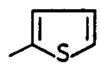 , 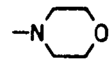 , 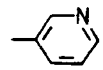 ,

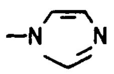 , 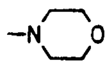 , 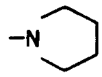 oder 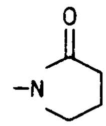

enthaltendes $C_1$-$C_{22}$-Alkyl, oder

- wenn n = 2 ist -

Y $C_2$- bis $C_{22}$-Alkylen, $C_5$- bis $C_{22}$-Cycloalkylen, $C_8$- bis $C_{14}$-Phenylalkylen, Phenylen oder durch Ethersauerstoff, Stickstoff, Schwefel oder 1,4-Piperazinylen unterbrochenes $C_4$- bis $C_{30}$-Alkylen bedeuten,

sowie die Säureadditionssalze dieser Verbindungen.

**2.** Piperidinderivate gemäß Anspruch 1, wobei in der Formel $R^1$, $R^2$, $R^3$ und $R^4$ Methyl bedeuten.

**3.** Piperidinderivate gemäß Anspruch 1 oder 2, wobei in der Formel $R^5$ Wasserstoff bedeutet.

**4.** Verwendung der Piperidinderivate gemäß den Ansprüchen 1 bis 3 zum Stabilisieren von organischem Material.

**5.** Verwendung der Piperidinderivate gemäß den Ansprüchen 1 bis 3 zum Stabilisieren von Polyolefinen.

**6.** Verwendung der Piperidinderivate gemäß den Ansprüchen 1 bis 3 zum Stabilisieren von Polyurethanen.

**7.** Verwendung der Piperidinderivate gemäß den Ansprüchen 1 bis 3 zum Stabilisieren von Polyamiden.

**8.** Stabilisiertes organisches Material, enthaltend mindestens ein Piperidinderivat gemäß Anspruch 1, 2 oder 3.

## Claims

**1.** A 4-formylaminopiperidine derivative of the formula (I)

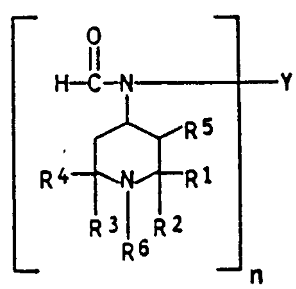 (I)

where n is 1 or 2, $R^1$, $R^2$, $R^3$ and $R^4$ independently of one another are each $C_1$-$C_4$-alkyl, or $R^1$ and $R^2$ or $R^3$ and $R^4$ together form a tetramethylene or pentamethylene group, $R^5$ is hydrogen or $C_1$-$C_4$-alkyl, $R^6$ is hydrogen, $C_1$-$C_{22}$-alkyl or $C_3$-$C_{22}$-alkenyl or is $C_7$-$C_{12}$-phenylalkyl which is unsubstituted or substituted by $C_1$-$C_4$-alkyl, fluorine, chlorine, $C_1$-$C_4$-alkoxy, methylenedioxy, ethylenedioxy and/or di-$C_1$-$C_4$-alkylamino or is $C_1$-$C_{22}$-alkanoyl, $C_2$- or $C_3$-cyanoalkyl, $C_1$-$C_{22}$-hydroxyalkyl or $C_2$-$C_{22}$-aminoalkyl and, when n is 1, Y is hydrogen, $C_1$-$C_{22}$-alkyl, $C_3$-$C_{22}$-alkenyl, $C_3$-$C_{12}$-cycloalkyl or bicycloalkyl or is $C_2$-$C_{22}$-alkyl which is substituted by cyano, hydroxyl or carbo-$C_1$-$C_4$-alkoxy, or is $C_4$-$C_{22}$-alkyl which is interrupted by ether oxygen, nitrogen or sulfur, or is $C_7$-$C_{22}$-phenyl- or diphenylalkyl which is unsubstituted or substituted by $C_1$-$C_4$-alkyl, fluorine, chlorine, $C_1$-$C_4$-alkoxy, methylenedioxy, ethylenedioxy or di-$C_1$-$C_4$-alkylamino, or is phenyl which is unsubstituted or substituted by $C_1$-$C_4$-alkyl

or carbo-$C_1$-$C_4$-alkoxy, or is a radical of the formula

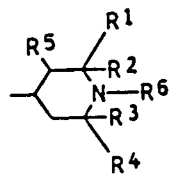

or $C_1$-$C_{22}$-alkyl containing heterocyclic radicals of the formula

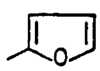 , 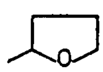 , 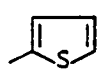 , 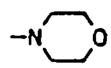 , 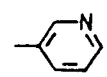 ,

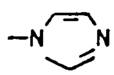 , 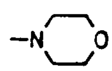 , 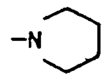 or 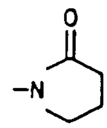

or, when n is 2, Y is $C_2$-$C_{22}$-alkylene, $C_5$-$C_{22}$-cycloalkylene, $C_8$-$C_{14}$-phenylalkylene or phenylene, or is $C_4$-$C_{30}$-alkylene which is interrupted by ether oxygen, nitrogen, sulfur or 1,4-piperazinylene, and the acid addition salts of this compound.

2. A piperidine derivative as claimed in claim 1, wherein, in the formula, $R^1$, $R^2$, $R^3$ and $R^4$ are each methyl.

3. A piperidine derivative as claimed in claim 1 or 2, wherein, in the formula, $R^5$ is hydrogen.

4. Use of a piperidine derivative as claimed in any of claims 1 to 3 for stabilizing organic material.

5. Use of a piperidine derivative as claimed in any of claims 1 to 3 for stabilizing polyolefins.

6. Use of a piperidine derivative as claimed in any of claims 1 to 3 for stabilizing polyurethanes.

7. Use of a piperidine derivative as claimed in any of claims 1 to 3 for stabilizing polyamides.

8. A stabilized organic material containing one or more piperidine derivatives as claimed in claim 1, 2 or 3.

**Revendications**

1. Dérivés de 4-formylaminopipéridine de formule générale (I)

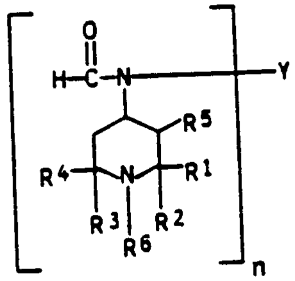 (I),

dans laquelle

n est mis pour 1 ou 2,

$R^1$, $R^2$, $R^3$ et $R^4$ représentent chacun, indépendamment les uns des autres, un radical alkyle en $C_1$ à $C_4$, ou

$R^1$ et $R^2$ ou $R^3$ et $R^4$ forment ensemble un groupement tétraméthylène ou pentaméthylène,

$R^5$ représente un atome d'hydrogène ou un radical alkyle en $C_1$ à $C_4$,

$R^6$ représente un atome d'hydrogène; un radical alkyle en $C_1$-$C_{22}$; alcényle en $C_3$-$C_{22}$; phénylalkyle en $C_7$-$C_{12}$ éventuellement substitué par des restes alkyle en $C_1$-$C_4$, fluoro, chloro, alcoxy, en $C_1$-$C_4$, methylènedioxy, éthylènedioxy et/ou di-$C_1$-$C_4$-alkylamino; alcanoyle en $C_1$-$C_{22}$; cyanoalkyle en $C_2$ ou $C_3$; hydroxyalkyle en $C_1$-$C_{22}$ ou aminoalkyle en $C_2$-$C_{22}$, et

lorsque n = 1,

Y représente un atome d'hydrogène; un radical alkyle en $C_1$-$C_{22}$; alcényle en $C_3$-$C_{22}$; cycloalkyle ou bicycloalkyle en $C_3$-$C_{22}$; alkyle en $C_2$-$C_{22}$ substitué par des restes cyano, hydroxy ou carbo(alcoxy en $C_1$-$C_4$);alkyle en $C_4$-$C_{22}$ interrompu par des atomes oxygène de type éther ou d'azote ou de soufre; phényl- ou ou diphénylalkyle en $C_7$-$C_{12}$ éventuellement substitué par des restes alkyle en $C_1$-$C_4$, fluoro, chloro, alcoxy en $C_1$-$C_4$, méthylènedioxy, éthylènedioy et/ou di(alkyl en $C_1$-$C_4$)amino; phényle éventuellement substitué par des restes alkyle en $C_1$-$C_4$ ou carbo(alcoxy en $C_1$-$C_4$); un radical de formule

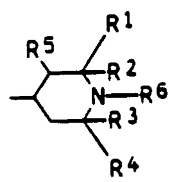

ou un radical alkyle en $C_1$-$C_{22}$ contenant des restes hétérocycliques de formule

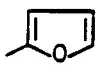 , 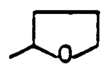 , 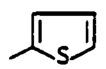 , 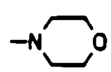 , 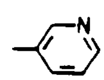

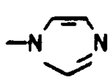 , 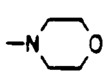 , 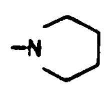 ou 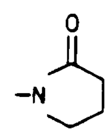

ou - lorsque n = 2 -

Y représente un radical alkylène en $C_2$-$C_{22}$,cycloalkylène en $C_5$-$C_{22}$, phénylalkylène en $C_8$-$C_{14}$, phénylène ou alkylène en $C_4$-$C_{30}$ interrompu par des atomes d'oxygène de type éther, d'azote ou de soufre ou par des groupements 1,4-pipérazinylène,

ainsi que les sels d'addition d'acides de ces composés.

**2.** Dérivés de pipéridine selon la revendication 1, dans la formule desquels $R^1$, $R^2$, $R^3$ et $R^4$ représentent des radicaux méthyle.

**3.** Dérivés de pipéridine selon la revendication 1 ou 2, dans la formule desquels $R^5$ représente un atome d'hydrogène.

**4.** Utilisation des dérives de pipéridine selon l'une quelconque des revendications 1 à 3 pour la stabilisation de matières organiques.

**5.** Utilisation des dérivés de pipéridine selon l'une quelconque des revendications 1 à 3 pour la stabilisation de polyoléfines.

**6.** Utilisation des dérivés de pipéridine selon l'une quelconque des revendications 1 à 3 pour la stabilisation de polyuréthannes.

7. Utilisation des dérivés de pipéridine selon l'une quelconque des revendications 1 à 3 pour la stabilisation de polyamides.

8. Matière organique stabilisée, contenant au moins un dérivé de pipéridine selon l'une quelconque des revendications 1 à 3.